# EUROPEAN PATENT APPLICATION

(11) **EP 0 737 477 A1**
(43) Date of publication of application: **16.10.1996**
(21) Application number: 95903976.9
(22) Date of filing: 26.12.1994
(51) Int. Cl.: A61K 31/57, A61K 9/70

(54) **PERCUTANEOUSLY ABSORBABLE PREPARATION**

(30) Priority: 27.12.1993 JP 333058/93; 27.12.1993 JP 333060/93
(71) Applicant: AKZO NOBEL N.V., 6800 SB Arnhem (NL)
(72) Inventor: KURODA, Hiroshi, Osaka 653 (JP); AZUMA, Masato, Osaka 569 (JP); HASHIMOTO, Masaki, Yamaguchi 746 (JP); WAKIYA, Takeshi, Seikacho Soraku-gun Kyoto 619-02 (JP); MANO, Mitsuhito, Hommachi Fushimi-ku Kyoto-shi Kyoto 612 (JP); KITAMURA, Mikiya, Osaka 571 (JP)
(74) Representative: Beetz, Tom
(86) International application number: JP9402237
(87) International publication number: WO9517896

(57) **Abstract**

A first percutaneously absorbable preparation comprises a base, at least one drug selected from the group consisting of 3-ketodesogestrel and 17-esters thereof, and/or at least one drug selected from the group consisting of 17-β-estradiol and esters thereof. A second percutaneously absorbable preparation comprises a support layer and, formed on one side thereof, a pressure-sensitive adhesive base layer comprising a pressure-sensitive adhesive, at least one drug selected from the group consisting of 3-ketodesogestrel and 17-esters thereof, and optionally at least one drug selected from the group consisting of 17-β-estradiol and esters thereof. These preparations are easy to produce and use, and can supply 3-ketodesogestrel or 17-esters thereof, and/or 17-β-estradiol or esters thereof through horny skins uniformly for long. Therefore they can be utilized for contraception, alleviation of menopause symptom, asteoporosis, menstruation disorder, and so forth.

## Description

### TECHNICAL FIELD

The present invention relates to a percutaneous pharmaceutical preparation.

3-Ketodesogestrel and its 17-esters belong to a kind of progestogen, and are known to be effective as a contraceptive and to relieve the symptoms of menopausal disorders. 17-β-estradiol and its esters belong to a kind of estrogen and are known to be effective in relieving the symptoms of menopausal disorders, osteoporosis, emmeniopathy, etc. In addition, it is also known that the combined administration of 17-β-estradiol and progestogen has a contraceptive effect and is effective in relieving the symptoms of menopausal disorders. (For instance, refer to Contraception, Vol. 17; 19-25 (1978).)

However, such progestogen has substantial side effects and, progestogen causes cerebral thrombosis, cerebral infarction, arteriosclerosis, stenocardia, myocardial infarction, uterine cancer, etc when a large amount of it is administered. On the other hand, estrogen also increases the risk of endometriosis, uterine cancer and others when it is continuously administered in large amounts, and also adversely affects the lipometabolic system, the hemocoagulative system, the cardiovascular system and others. In particular, there is a possibility that estrogen causes cholecystitis, cholecystolithiasis and others, when it is administered orally. Furthermore, it is defective in that its peroral administration is much less efficacious than its administration by injection.

In order to overcome these serious drawbacks, various methods for preparing slow-release drugs have been reported. For instance, in Japanese Patent Application Laid-Open No. 64-70410, an implantation device has been proposed for subcutaneous application or local application, which is ethylene-vinyl acetate copolymer film coating core material composed of ethylene-vinyl acetate copolymer and 3-ketodesogestrel.

In Japanese Patent Application Laid-Open No. 61-106508, a vaginal ring release system is described comprising a two-partitioned tubular ring made of a porous film of a polymer such as polysiloxane, polyurethane and each partition of the ring contains progestogen and estrogen separately.

However, the above-mentioned implant shall be either subcutaneously implanted or locally applied into the uterus or into the region of the cervical canal. The subcutaneous implantation was not economically feasible in that it required a doctor to perform the implantation which is costly and time-consuming. The local application was also disadvantageous in that it required much time, and, after the application, the patient felt discomfort.

The latter vaginal ring release system was disadvantageous in that the preparation of the system is difficult and that much time is needed to introduce it into the vagina and, in addition, the patient will experience some degree of discomfort during its use.

The object of the present invention is to provide a percutaneous pharmaceutical preparation which is easy to produce, is easy to use and provide 3-ketodesogestrel and its 17-ester or a combinaiton of 3-ketodesogestrel and its 17-ester and 17-β-estradiol and its ester for a prolonged periods through the skin with the stratum corneum.

### DESCRIPTION OF THE INVENTION

The first percutaneous pharmaceutical preparation of the present invention comprises a base material, one or more active ingredients selected from a group consisting of 3-ketodesogestrel and its 17-esters, and/or one or more active ingredients selected from a group consisting of 17-β-estradiol and its esters.

The second percutaneous pharmaceutical preparation of the present invention is composed of a backing layer and an adhesive base layer laminated on one surface of the backing layer, in which the adhesive base layer comprises an adhesive, one or more active ingredients selected from a group consisting of 3-ketodesogestrel and its 17-esters, and optionally one or more active ingredients selected from a group consisting of 17-β-estradiol and its esters.

The first percutaneous pharmaceutical preparation is described below.

The active ingredients used in the present invention are 3-ketodesogestrel and its 17-esters, and/or 17-β-estradiol and its esters. The esters of 17-β-estradiol may be either mono-esters or di-esters. The esters of 3-ketodesogestrel and 17-β-estradiol are obtained by esterifying 3-ketodesogestrel and 17-β-estradiol, respectively, with acids. The acids are organic monocarboxylic acids such as acetic acid, valeric acid, benzoic acid, propionic acid, cipionic acid, undecylenic acid, enanthic acid, etc.

The first percutaneous pharmaceutical preparation of the present invention comprises the above-mentioned base material and one or more active ingredients selected from a group consisting of 3-ketodesogestrel and its 17-esters, and/or one or more active ingredients selected from a group consisting of 17-β-estradiol and its esters. If the amount of the active ingredient(s) in the first preparation is small, the preparation will be inefficacious, but if it is too large, the active ingredient(s) will precipitate out of the base material. Therefore, the optimal preparation amount of the active ingredient(s) is from 0.001 to 20 % by weight, more preferably from 0.05 to 15 % by weight, especially preferably from 0.1 to 10 % by weight, of the percutaneous pharmaceutical preparation in order that the excess amount of active ingredient(s) does not precipitate.

In the percutaneous pharmaceutical preparation containing both one or more active ingredients selected from a group consisting of 3-ketodesogestrel and its 17-esters, and one or more active ingredients selected from a group consisting of 17-β-estradiol and its esters, the ratio of the former pharmaceutical component(s) to the latter pharmaceutical component(s) by weight is not specifically defined, but in general, it is from 1:10 to 10:1, and preferably from 1:5 to 5:1.

The base material used in the first percutaneous pharmaceutical preparation may include any of the pharmaceutically acceptable ones that may dissolve the above-mentioned active ingredients. Usable are those that have heretofore been used as the base material for ointments, creams, jellies, pastes, lotions, etc. For instance, mentioned are polymers such as sodium alginate, gelatin, corn starch, tragacanth gum, methyl cellulose, hydroxyethyl cellulose, hydroxypropyl cellulose, carboxymethyl cellulose, sodium carboxymethyl cellulose, dextrin, carboxymethyl starch, polyvinyl alcohol, polyacrylic acid, polyacrylates (e.g., sodium polyacrylate), methoxyethylene-maleic anhydride copolymer, polyvinyl ether, polyvinyl pyrrolidone, etc.; colloidal aluminium silicate hydrate minerals such as kaolin, bentonite, etc., colloidal magnesium aluminium silicate hydrate minerals such as bee gum, etc.; oils and fats such as bees wax, olive oil, cacao oil, sesame oil, soybean oil, camellia oil, peanut oil, beef tallow, lard, lanolin, vaseline, etc.; white vaseline; paraffins; liquid paraffin; plastibase; polyalcohols such as glycerin, ethylene glycol, polyethylene glycol, propylene glycol, polypropylene glycol, etc.; higher fatty acids such as stearic acid, etc.; lower alcohols such as isopropyl alcohol, ethyl alcohol, etc.; higher alcohols such as cetyl alcohol, stearyl alcohol, etc.; water, etc. These are used as they are or as compositions containing one or more of them.

If desired, the base material may contain an inorganic filler such as silica, zinc oxide, titanium oxide, etc.; a moisturizing agent such as sorbitol, etc.; a viscosity-adjusting agent; an antioxidant; a pH-adjusting agent, etc.

The first percutaneous pharmaceutical preparation may contain, if desired, a percutaneous absorption accelerating agent. As examples of the percutaneous absorption accelerating agent usable in the present invention, mentioned are N-acylsarcosines or their salts; monocarboxylic acids having from 8 to 14 carbon atoms or their salts; dicarboxylic acids having from 2 to 8 carbon atoms or their salts; hydroxydicarboxylic acids having from 3 to 8 carbon atoms; fatty acid amides, or reaction products of aliphatic monocarboxylic acid(s) having from 8 to 16 carbon atoms and mono- or di-ethanolamine(s); and esters of higher fatty acids, or reaction products of higher fatty acid(s) having from 10 to 18 carbon atoms and alcohol(s) having from 1 to 18 carbon atoms. These can be used singly or as a combination of two or more of them.

The N-acylsarcosines or their salts include, for example, N-lauroylsarcosine, N-stearoylsarcosine, N-oleoylsarcosine, N-palmitoylsarcosine, coconut-oil fatty acid sarcosines, etc., and their salts such as sodium, potassium, magnesium, calcium and aluminium salts of these N-acylsarcosines.

The monocarboxylic acids or their salts are specified to have from 8 to 14 carbon atoms, because, if they have 7 or fewer carbon atoms, they are so strongly acidic that they cannot be administered to the human body, and if they have 15 or more carbon atoms, the absorption accelerating effect will be lowered. Therefore, it is specified that the carbon number of the acids falls within the range of from 8 to 14. Preferred examples of these include saturated aliphatic linear monocarboxylic acids such as capric acid, nonanoic acid, caprylic acid, lauric acid, myristic acid and the like, and their salts such as sodium, potassium, magnesium, calcium and aluminium salts of these monocarboxylic acids.

It is specified that the dicarboxylic acids or their salts have from 2 to 8 carbon atoms, because, if they have 9 or more carbon atoms, their absorption accelerating effect is lowered. Therefore, the carbon number of the acids is to fall within the range of from 2 to 8. Preferred examples of these include saturated aliphatic linear dicarboxylic acids such as oxalic acid, malonic acid, succinic acid, glutaric acid, pimelic acid, suberic acid, etc.; unsaturated aliphatic linear dicarboxylic acids such as fumaric acid, maleic acid, etc.; aromatic dicarboxylic acids such as phthalic acid, isophthalic acid, terephthalic acid, etc.; and their salts such as sodium, potassium, magnesium, calcium and aluminium salts of these dicarboxylic acids.

It is specified that the hydroxydicarboxylic acids or their salts have from 3 to 8 carbon atoms, because, if they have 9 or more carbon atoms, their absorption accelerating effect will be lowered. Therefore, the carbon number of the acids is to fall within the range of from 3 to 8. Preferred examples of these include malic acid, tartaric acid, and their salts such as their sodium, potassium, magnesium, calcium and aluminium salts.

The fatty acid amides are the reaction products of aliphatic monocarboxylic acid(s) having from 8 to 16 carbon atoms and mono- or di-ethanolamine(s). Aliphatic monocarboxylic acids having 7 or fewer carbon atoms are volatile, while those having 17 or more carbon atoms are less effective in promoting the absorbability of the active ingredients. Therefore, the acids are specified to have from 8 to 16 carbon atoms. For instance, they are saturated aliphatic monocarboxylic acids, including lauric acid, palmitic acid, myristic acid, capric acid, caprylic acid, etc. Lauric acid diethanolamide is especially preferred in the fatty acid amide.

The higher fatty acids are specified to have from 10 to 18 carbon atoms, because, if they have 9 or fewer carbon atoms, they are volatile, but if they have 19 or more carbon atoms, the absorption accelerating effect of the accelerating agent is lowered. For instance, the acids include saturated aliphatic monocarboxylic acids such as lauric acid, palmitic acid, myristic acid, capric acid, stearic acid, etc.; unsaturated aliphatic monocarboxylic acids such as palmitoleic acid, oleic acid, vaccenic acid, linolic acid, linolenic acid, etc.; and saturated aliphatic dicarboxylic acids such as sebacic acid, etc. The higher fatty acids may be employed singly or as a combination of two or more.

The alcohols are specified to have from 1 to 18 carbon atoms, because, if they have 19 or more carbon atoms, the absorption accelerating effect of the accelerating agent is lowered. For instance, the alcohols are preferably aliphatic saturated alcohols such as methyl alcohol, ethyl alcohol, propyl alcohol, isopropyl alcohol, butyl alcohol, pentyl alcohol, hexyl alcohol, heptyl alcohol, octyl alcohol, decyl alcohol, cetyl alcohol, etc. The alcohols may be employed singly or as a combination of two or more.

The esters of higher fatty acids are preferably isopropyl myristate and isopropyl palmitate.

If the amount of the above-mentioned percutaneous absorption accelerating agent to be added to the first percutaneous pharmaceutical preparation is small, the absorption accelerating effect of the accelerating agent is lowered, but if it is too large, the compatibility of the accelerating agent with the base material is lowered. Therefore, the amount is preferably from 0.1 to 20 % by weight, more preferably from 1 to 15 % by weight, especially preferably from 3 to 10 % by weight, of the percutaneous pharmaceutical preparation.

The first percutaneous pharmaceutical preparation which constitution is mentioned above is obtained by mixing and dissolving the active ingredients and optionally the absorption accelerating agents on the base material. Preferably, it is formulated by ordinary methods of formulating medicines into ointments, creams, jellies, pastes, lotions and the like. To produce an endermic cataplasm, a layer containing the base material, the pharmaceutical component(s) and, optionally, the absorption accelerating agent(s) is laminated over one surface of a backing made of, for example, cellulose acetate, ethyl cellulose, polyethylene terephthalate, vinyl acetate-vinyl chloride copolymer, ethylene-vinyl acetate copolymer, polyvinyl chloride, polyvinylidene chloride, nylon, polyurethane, aluminium, non-woven fabric, woven fabric and the like.

As the formulation of the first percutaneous pharmaceutical preparation, ointment is preferred.

As the base material for the ointment, preferred is a gel comprising a polyacrylic acid or its salt, a polyethylene glycol or propylene glycol, a lower alcohol (for example, ethyl alcohol, isopropyl alcohol, etc.) and water.

The amount of the polyacrylic acid or its salt is preferably from 0.01 to 3 % by weight, more preferably from 0.5 to 2.3 % by weight, of the ointment.

The amount of the polyethylene glycol or propylene glycol is preferably from 5 to 45 % by weight, more preferably from 10 to 44 % by weight, of the ointment.

The amount of the lower alcohol is preferably from 20 to 65 % by weight, more preferably from 25 to 64 % by weight, of the ointment.

The amount of water is preferably from 5 to 30 % by weight, more preferably from 10 to 28 % by weight, of the ointment.

As the percutaneous absorption accelerating agent to be added to the ointment, preferred are fatty acid amides, and especially preferred is lauric acid diethanolamide. The amount of lauric acid diethanolamide is preferably from 0.1 to 20 % by weight, more preferably from 1 to 15 % by weight, especially preferably from 3 to 10 % by weight, of the ointment.

A preferred embodiment comprises a percutaneous pharmaceutical preparation comprising polyacrylic acid as base and lauric acid diethanolamide as percutaneous absorption accelerating agent. Such preparations from a harder gel, having less tendency to spread out over the body's surface.

The above-mentioned preparation comprises a steroid as the active ingredient. The active ingredient is preferably a progestagenic compound. More preferably, the active ingredient is a 19-nor-testosterone derivative. The 19-nor-testosterone derivative is preferably selected from a group consisting of desogestrel, 3-ketodesogestrel, levonorgestrel, gestodene, norgestrel, norgestrienone, noretistherone, norethindron, norgestimate, and esters thereof. Especially preferred is 3-ketodesogestrel or an ester thereof. It is preferred that the active ingredient in the above-mentioned preparation further comprises an estrogenic compound. It is preferred that the estrogenic compound is selected from a group consisting of ethynyl estradiol, β-estradiol, and esters thereof. The preparation is preferably a percutaneous pharmaceutical preparation comprising an active ingredient selected from a group consisting of 3-ketodesogestrel, β-estradiol, and esters thereof.

The amount of the lauric acid diethanolamide in the above-mentioned preparation is preferably from 0.1 to 20% by weight, more preferably from 3 to 10% by weight, of the preparation.

Next, the second percutaneous pharmaceutical preparation of the present invention will be described below, which is composed of a backing layer and an adhesive base layer laminated on one surface of the backing layer, in which the adhesive base layer comprises an adhesive, one or more active ingredients selected from a group consisting of 3-ketodesogestrel and its 17-esters, and optionally one or more active ingredients selected from a group consisting of 17-β-estradiol and its esters.

The support material (backing layer) to be used in the second percutaneous pharmaceutical preparation is preferably soft and drug-impermeable or hardly drug-permeable. For instance, it may include resin films of, for example, cellulose acetate, ethyl cellulose, polyethylene, polypropylene, polyvinyl chloride, vinyl acetate-vinyl chloride copolymer, ethylene-vinyl acetate copolymer, polyvinylidene chloride, polyurethane, nylon, polyethylene terephthalate, polybutylene terephthalate, ethylene-vinyl acetate-carbon monoxide copolymer, ethylene-butyl acrylate-carbon monoxide copolymer or the like, as well as aluminium sheeting. Laminate sheets of these resin film may also be used, or they may be laminated on woven or non-woven fabric.

The pharmaceutical components to be in the second percutaneous pharmaceutical preparation are the same as those mentioned for the first percutaneous pharmaceutical preparation.

The second percutaneous pharmaceutical preparation contains one or more active ingredients selected from a group consisting of 3-ketodesogestrel and its 17-esters, and optionally one or more active ingredients selected from a group consisting of 17-β-estradiol and its esters. If the amount of the active ingredient(s) in the preparation is small, the preparation will be inefficacious, but if it is too large, the component(s) will precipitate out of the base material. Therefore, the optimal preparation amount of the active ingredient(s) is from 0.001 to 20 % by weight, more preferably from 0.1 to 15 % by weight, especially preferably from 0.5 to 12 % by weight, in order that the excess amount of active gredient(s) does not precipitate.

In the percutaneous pharmaceutical preparation containing both one or more active ingredients selected from a group consisting of 3-ketodesogestrel and its 17-esters, and one or more active ingredients selected from a group consisting of 17-β-estradiol and its esters, the ratio of the former pharmaceutical component(s) to the latter pharmaceutical component(s) by weight is not specifically defined, but in general, it is from 1:10 to 10:1, and preferably from 1:5 to 5:1.

The adhesive to be used in the second percutaneous pharmaceutical preparation may be of any of the pharmaceutically acceptable ones. Any conventional known adhesives may be used, including, for example, acrylic adhesives, rubber adhesives, silicone adhesives, urethane adhesives, etc. It may be of the solvent type, emulsion type, hot-melt type or other type.

The acrylic adhesives are those consisting essentially of alkyl (meth)acrylates. They may also be copolymers comprising alkyl (meth)acrylates and copolymerizable functional monomers or polyfunctional monomers.

As alkyl (meth)acrylates, mentioned are methyl (meth)acrylate, ethyl (meth)acrylate, butyl (meth)acrylate, 2-ethylhexyl (meth)acrylate, n-octyl (meth)acrylate, dodecyl (meth)acrylate, etc. The number of the carbon atoms of the alkyl group of the acrylates is preferably from 4 to 12, because, if it is small, the adhesive strength of the adhesive is lowered though the cohesive strength thereof increase, but if the number of the carbon atoms is large, the cohesive strength is lowered while the adhesive strength increase.

As functional monomers, mentioned are (meth)acrylic acid, 2-hydroxyethyl (meth)acrylate, 2-hydroxypropyl (meth)acrylate, glycidyl methacrylate, N-methylol(meth)acrylamide, N-butoxymethylacrylamide, etc.

Copolymers obtainable by copolymerization with such functional monomers may be crosslinked with metal salts such as sodium hydroxide, calcium hydroxide or the like or with isocyanates, epoxy resins, melamine resins, urea resins, ammoniums or the like, thereby elevating the cohesive force of the adhesives.

Polyfunctional monomers are copolymerized for the purpose of elevating the cohesive strength of the adhesives. They include, for example, 1,6-hexaneglycol dimethacrylate, tetraethylene-glycol diacrylate, trimethylolpropane triacrylate, divinylbenzene, divinyltoluene, diallyl phthalate, diallyl maleate, diallyl adipate, diallyl glycolate, triallyl isocyanurate, diethylene-glycol bisallylcarbonate, etc.

The acrylic adhesive may be copolymer comprised alkyl (meth)acrylate monomer and vinyl compounds which can be copolymerized with alkyl (meth)acrylate monomer. As such vinyl compounds, mentioned are, for example, vinyl acetate, acrylonitrile, styrene, N-vinyl-2-pyrrolidone, etc.

Especially preferred are acrylic adhesives comprising copolymers composed of from 55 to 95 % by weight of 2-ethylhexyl acrylate, from 5 to 45 % by weight of N-vinyl-2-pyrrolidone and from 0 to 0.5 % by weight of polyfunctional monomer(s).

The acrylic adhesives may comprise copolymers composed of plural alkyl (meth)acrylate monomers.

Especially preferred are acrylic adhesives comprising copolymers composed of from 65 to 90 % by weight of 2-ethylhexyl methacrylate, from 5 to 30 % by weight of 2-ethylhexyl acrylate, from 5 to 30 % by weight of dodecyl methacrylate and from 0 to 0.5 % by weight of polyfunctional monomer(s).

The acrylic adhesives consist essentially of alkyl (meth)acrylates and other suitable constitutive components may be employed, depending upon the necessary properties. In general, the functional monomers are copolymerized in an amount of 20 % by weight or less, preferably from 1 to 10 % by weight, of the adhesive. The polyfunctional monomers are copolymerized generally in an amount of from 0 to 0.5 % by weight of the adhesive. The vinyl compounds are copolymerized generally in an amount of 50 % by weight or less, preferably 40 % by weight or less, of the same.

Needless-to-say, the acrylic adhesives may contain a tackifier, a filler and other components, in such amounts that they are pharmaceutically acceptable.

The rubber adhesives contain a rubber material such as, for example, natural rubber, styrene-butadiene rubber, polyisobutylene, styrene-isoprene-styrene block copolymer, styrene-butadiene-styrene block copolymer, styrene-olefin-styrene block copolymer, etc. In general, they contain a tackifier such as, for example, rosin, hydrogenated rosin, rosin esters, terpene resins, terpene-phenol resins, petroleum resins, coumarone resins, coumarone-indene resins, etc.

Further, they may also contain oils such as almond oil, olive oil, camellia oil, peanut oil, sesame oil, soybean oil, mink oil, cotton seed oil, corn oil, safflower oil, coconut oil, castor oil, etc.; and oleic acid, liquid paraffin and others.

The second percutaneous pharmaceutical preparation may also contain, if desired, a percutaneous absorption accelerating agent in its adhesive base layer. The percutaneous absorption accelerating agent to be in this may be the same as that referred to hereinabove for the first percutaneous pharmaceutical preparation.

Regarding the amount of the percutaneous absorption accelerating agent to be added to the second percutaneous pharmaceutical preparation, if it is small, the absorption accelerating effect of the accelerating agent is lowered, but if it is too large, the compatibility of the accelerating agent with the base material is lowered. Therefore, the amount is preferably from 0.1 to 20 % by weight, more preferably from 1 to 15 % by weight, especially preferably from 3 to 10 % by weight, of the adhesive base layer.

The thickness of the adhesive base layer is not specifically defined, but if it is too thin, large amounts of the active ingredients must be added thereto which results in that the adhesive strength of the layer is lowered, but if it is too thick, the active ingredients in the pharmaceutical preparation cannot be utilized effectively while the cost of the pharmaceutical preparation is elevated without improving its properties. Therefore, the thickness is preferably from 20 to 200 µm, more preferably from 30 to 100 µm.

The second percutaneous pharmaceutical preparation of which constitution is as mentioned above, which may be produced by any of the desired methods. For instance, employable are a method of blending a solvent type or emulsion type adhesive and the active ingredient(s) optionally along with the absorption accelerating agent followed by coating the resulting blend on a support material and drying it; a method of coating the blend on a release paper, drying it, and transferring it onto a support material; and a method of blending a hot-melt type adhesive and the active ingredient(s) optionally along with the absorption accelerating agent followed by coating the resulting blend on a support material.

3-Ketodesogestrel and its 17-esters are relatively unstable. In particular, when they are together with acids, they decompose with the time. Therefore, in storing the second percutaneous pharmaceutical preparation, it is preferred that the preparation is shielded from oxygen or is stored together with a disoxidant. Preferably, the percutaneous pharmaceutical preparation of the present invention is wrapped with a film having only slight oxygen permeability, especially having an oxygen permeability of 20 cm³/m².atm.24 hrs. (25°C) or less. As examples of such a film, mentioned are aluminium sheeting, polyvinylidene chloride film, polyvinylidene chloride-polyethylene laminate film, polyvinylidene chloride-polypropylene laminate film, polyvinylidene chloride-polyvinyl chloride laminate film, polyvinyl alcohol film, nylon film, etc.

The disoxidant can absorb or remove oxygen and may be any of the known ones. For instance, usable are powders, pills, granules or tablets of active iron oxide, hydrosulfite (sodium dithionite), ascorbic acid, butylhydroxy-toluene or the like. Known are various commercial products such as Ageless Z-20 (produced by Mitsubishi Gas Chemical Co.), Sendo-hojizai F (preservative, produced by Toppan Printing Co.), etc.

The constitutions of the first and second percutaneous pharmaceutical preparations of the present invention are as mentioned above. These preparations can be produced easily and can be used easily, while percutaneously releasing 3-ketodesogestrel or its 17-ester, and/or 17-β-estradiol and its ester, uniformly over a prolonged period of time through the skin with the stratum corneum.

The percutaneous pharmaceutical preparations containing a percutaneous absorption accelerating agent in its base material or its adhesive base layer provide better percutaneous absorbability.

Accordingly, the percutaneous pharmaceutical preparations of the present invention can be used efficaciously for contraception and also for relieving the symptoms of menopausal disorders, osteoporosis, emmeniopathy, etc.

### BRIEF DESCRIPTION OF THE DRAWINGS

Fig. 1 shows the graphs of the results from the permeation tests (for 3-ketodesogestrel) in Examples 1 to 4.

Fig. 2 shows the graphs of the results of the concentration of 3-ketodesogestrel in blood in Examples 1 to 4 and Comparative Example 1.

Fig. 3 shows the graphs of the results from the permeation tests (for 3-ketodesogestrel) in Examples 5 to 9.

Fig. 4 shows the graphs of the results from the permeation tests (for 3-ketodesogestrel) in Examples 10 to 14.

Fig. 5 shows the graphs of the results from the permeation tests (for 3-ketodesogestrel) in Examples 15 to 19.

Fig. 6 shows the graphs of the results from the permeation tests (for 3-ketodesogestrel) in Examples 20 to 24.

Fig. 7 shows the graphs of the results of the concentration of 3-ketodesogestrel in blood in Examples 10, 11, 15, 16, 20 and 21.

Fig. 8 shows the graphs of the results from the permeation tests (for 3-ketodesogestrel) in Examples 25 to 28.

Fig. 9 shows the graphs of the results from the permeation tests (for 17-β-estradiol) in Examples 25 to 28.

Fig. 10 shows the graphs of the results of the concentration of 3-ketodesogestrel in blood in Examples 25 to 28 and Comparative Example 2.

Fig. 11 shows the graphs of the results of the concentration of 17-β-estradiol in blood in Examples 25 to 28 and Comparative Example 2.

Fig. 12 shows the graphs of the results from the permeation tests (for 3-ketodesogestrel) in Examples 29 to 33.

Fig. 13 shows the graphs of the results from the permeation tests (for 17-β-estradiol) in Examples 29 to 33.

Fig. 14 shows the graphs of the results from the permeation tests (for 3-ketodesogestrel) in Examples 34 to 38.

Fig. 15 shows the graphs of the results from the permeation tests (for 17-β-estradiol) in Examples 34 to 38.

Fig. 16 shows the graphs of the results from the permeation tests (for 3-ketodesogestrel) in Examples 39 to 43.

Fig. 17 shows the graphs of the results from the permeation tests (for 17-β-estradiol) in Examples 39 to 43.

Fig. 18 shows the graphs of the results from the permeation tests (for 3-ketodesogestrel) in Examples 44 to 48.

Fig. 19 shows the graphs of the results from the permeation tests (for 17-β-estradiol) in Examples 44 to 48.

Fig. 20 shows the graphs of the results of the concentration of 3-ketodesogestrel in blood in Examples 34, 39 and 44.

Fig. 21 shows the graphs of the results of the concentration of 17-β-estradiol in blood in Examples 34, 39 and 44.

Fig. 22 shows the graphs of the results from the permeation tests (for 17-β-estradiol) in Examples 49 to 52.

Fig. 23 shows the graphs of the results from the permeation tests (for 17-β-estradiol) in Examples 53 to 56.

Fig. 24 shows the graphs of the results from the permeation tests (for 17-β-estradiol) in Examples 57 to 61.

Fig. 25 shows the graphs of the results from the permeation tests (for 17-β-estradiol) in Examples 62 to 65, 68 and 77.

Fig. 26 shows the graphs of the results from the permeation tests (for 17-β-estradiol) in Examples 66, 67 and 69 to 71.

Fig. 27 shows the graphs of the results from the permeation tests (for 17-β-estradiol) in Examples 72 to 76.

Fig. 28 shows the graphs of the results from the permeation tests (for 17-β-estradiol) in Examples 92, 93, 96 and 105.

Fig. 29 shows the graphs of the results from the permeation tests (for 17-β-estradiol) in Examples 94, 95, 97 and 99.

Fig. 30 shows the graphs of the results from the permeation tests (for 17-β-estradiol) in Examples 100 to 104.

Fig. 31 shows the graphs of the results from the permeation tests (for 17-β-estradiol) in Examples 98 and 106 to 108.

Fig. 32 shows the graphs of the results from the permeation tests (for 3-ketodesogestrel) in Examples 78, 79, 82 and 91.

Fig. 33 shows the graphs of the results from the permeation tests (for 3-ketodesogestrel) in Examples 80, 81 and 83 to 85.

Fig. 34 shows the graphs of the results from the permeation tests (for 3-ketodesogestrel) in Examples 86 to 90.

Fig. 35 shows the graphs of the results from the permeation tests (for 3-ketodesogestrel) in Examples 92, 93, 96 and 105.

Fig. 36 shows the graphs of the results from the permeation tests (for 3-ketodesogestrel) in Examples 94, 95, 97 and 99.

Fig. 37 shows the graphs of the results from the permeation tests (for 3-ketodesogestrel) in Examples 100 to 104.

Fig. 38 shows the graphs of the results from the permeation tests (for 3-ketodesogestrel) in Examples 98 and 106 to 108.

Fig. 39 shows the graphs of the results of the concentration of 17-β-estradiol in blood in Examples 49 to 53, 57 and 62.

Fig. 40 shows the graphs of the results from the permeation tests (for 17-β-estradiol) in Examples 70, 72, 74, 98, 100 and 102, from which the comparative influence of the concentration of the polyacrylic acid on the percutaneous permeation is known.

Fig. 41 shows the graphs of the results from the permeation tests (for 3-ketodesogestrel) in Examples 84, 86, 88, 98, 100 and 102, from which the comparative influence of the concentration of the polyacrylic acid on the percutaneous permeation is known.

Fig. 42 shows the graphs of the results from the permeation tests (for 17-β-estradiol) in Examples 67, 68, 73, 95, 96 and 101, from which the comparative influence of the concentration of polyethylene glycol #400 on the percutaneous permeation is known.

Fig. 43 shows the graphs of the results from the permeation tests (for 3-ketodesogestrel) in Examples 81, 82, 87, 95, 96 and 101, from which the comparative influence of the concentration of polyethylene glycol #400 on the percutaneous permeation is known.

Fig. 44 shows the graphs of the results from the permeation tests (for 17-β-estradiol) in Examples 70, 75, 76, 98, 103 and 104, from which the comparative influence of the concentration of propylene glycol on the percutaneous permeation is known.

Fig. 45 shows the graphs of the results from the permeation tests (for 3-ketodesogestrel) in Examples 84, 89, 90, 98, 103 and 104, from which the comparative influence of the concentration of propylene glycol on the percutaneous permeation is known.

Fig. 46 shows the graphs of the results from the permeation tests (for 17-β-estradiol) in Examples 66, 68, 69, 94, 96 and 97, from which the comparative influence of the concentration of water or ethyl alcohol on the percutaneous permeation is known.

Fig. 47 shows the graphs of the results from the permeation tests (for 3-ketodesogestrel) in Examples 80, 82, 83, 94, 96 and 97, from which the comparative influence of the concentration of water or ethyl alcohol on the percutaneous permeation is known.

Fig. 48 shows the graphs of the results from the permeation tests (for 17-β-estradiol) in Examples 68, 71, 96 and 99, from which the comparative influence of ethyl alcohol and isopropyl alcohol on the percutaneous permeation is known.

Fig. 49 shows the graphs of the results from the permeation tests (for 3-ketodesogestrel) in Examples 82, 85, 96 and 99, from which the comparative influence of ethyl alcohol and isopropyl alcohol on the percutaneous permeation is known.

Fig. 50 shows the graphs of the results from the permeation tests (for 17-β-estradiol) in Examples 68, 70, 96 and 98, from which the comparative influence of polyethylene glycol #400 and propylene glycol on the percutaneous permeation is known.

Fig. 51 shows the graphs of the results from the permeation tests (for 3-ketodesogestrel) in Examples 82, 84, 96 and 98, from which the comparative influence of polyethylene glycol #400 and propylene glycol on the percutaneous permeation is known.

Fig. 52 shows the graphs of the results from the permeation tests (for 3-ketodesogestrel) in Examples 109 to 116.

Fig. 53 shows the graphs of the results from the permeation tests (for 3-ketodesogestrel) in Examples 117 to 122.

Fig. 54 shows the graphs of the results from the permeation tests (for 3-ketodesogestrel) in Examples 123 to 130.

Fig. 55 shows the graphs of the results from the permeation tests (for 3-ketodesogestrel) in Examples 131 to 138.

Fig. 56 shows the graphs of the results from the permeation tests (for 3-ketodesogestrel) in Examples 139 to 144.

Fig. 57 shows the graphs of the results from the permeation tests (for 3-ketodesogestrel) in Examples 145 to 152.

Fig. 58 shows the graphs of the results from the permeation tests (for 17-β-estradiol) in Examples 131 to 138.

Fig. 59 shows the graphs of the results from the permeation tests (for 17-β-estradiol) in Examples 139 to 144.

Fig. 60 shows the graphs of the results from the permeation tests (for 17-β-estradiol) in Examples 145 to 152.

Fig. 61 shows the graphs of the results of the concentration of 3-ketodesogestrel in blood in Examples 109, 117, 118, 120, 122 to 124, 128 and 129.

Fig. 62 shows the graphs of the results of the concentration of 3-ketodesogestrel in blood in Examples 131, 139, 140, 142, 144 to 146, 150 and 151.

Fig. 63 shows the graphs of the results of the concentration of 17-β-estradiol in blood in Examples 131, 139, 140, 142, 145 and 146.

Fig. 64 shows the graphs of the results of the concentration of 17-β-estradiol in blood in Examples 144, 150 and 151.

Fig. 65 is a perspective view of a diffusion cell.

### BEST MODES FOR CARRYING OUT THE INVENTION

Examples of the present invention are mentioned below.

### Examples 1 to 48:

Pre-determined amounts (parts by weight) of a base material [Plastibase (produced by Taisho Pharmaceutical Co.), bentonite magma (dispersion comprising 10 parts by weight of bentonite, 10 parts by weight of glycerin and 80 parts by weight of water), Macrogol ointment (mixture comprising 50 parts by weight of polyethylene glycol 400 (mean molecular weight) and 50 parts by weight of polyethylene glycol 4000 (mean molecular weight), or polyacrylic acid gel [polyacrylic acid gel D comprising pre-determined amounts (parts by weight) of polyacrylic acid, water, polyethylene glycol #400 (mean molecular weight), propylene glycol, ethyl alcohol and isopropyl alcohol, as indicated in Table 5]; active ingredient (3-ketodesogestrel and/or 17-β-estradiol); and percutaneous absorption accelerating agent(s) (N-lauroylsarcosine, maleic acid, malic acid, capric acid, lauric acid diethanolamide and/or isopropyl myristate), as indicated in Tables 1 to 4 below, were put in a mortar and stirred therein until all additives were dissolved in the base material to obtain samples of a percutaneous pharmaceutical preparation.

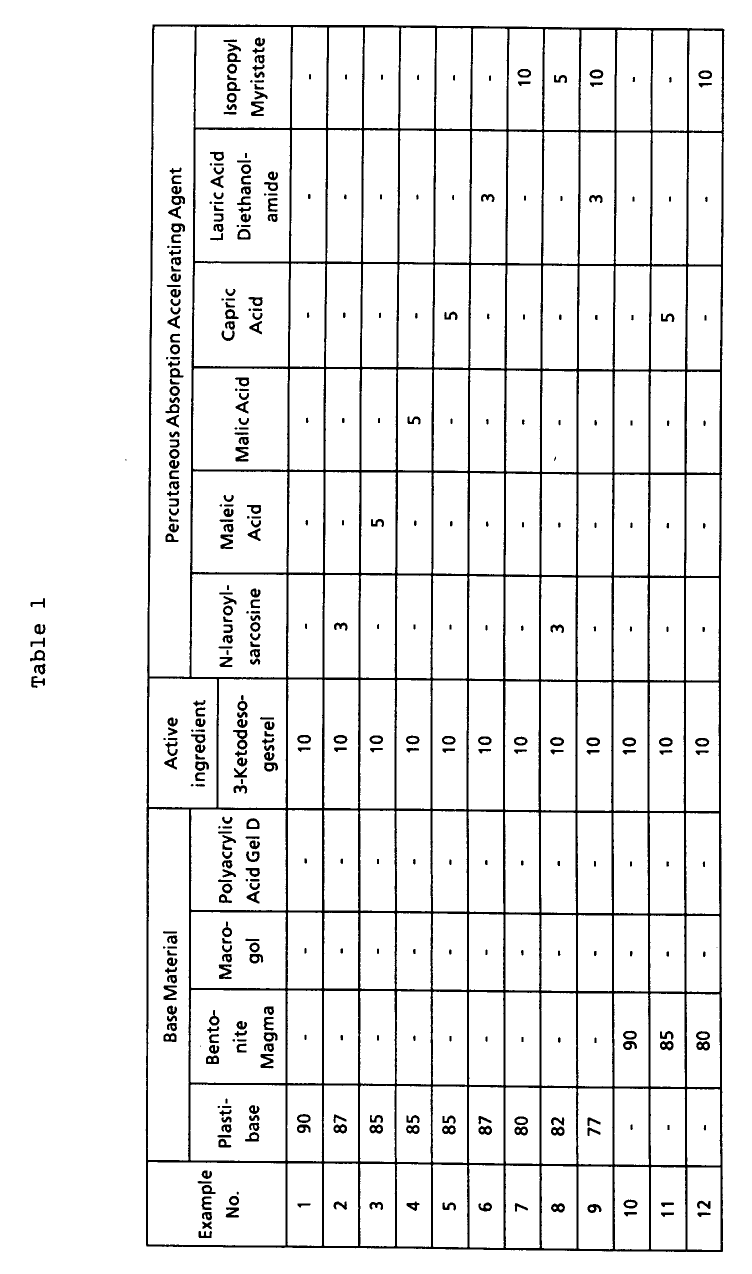

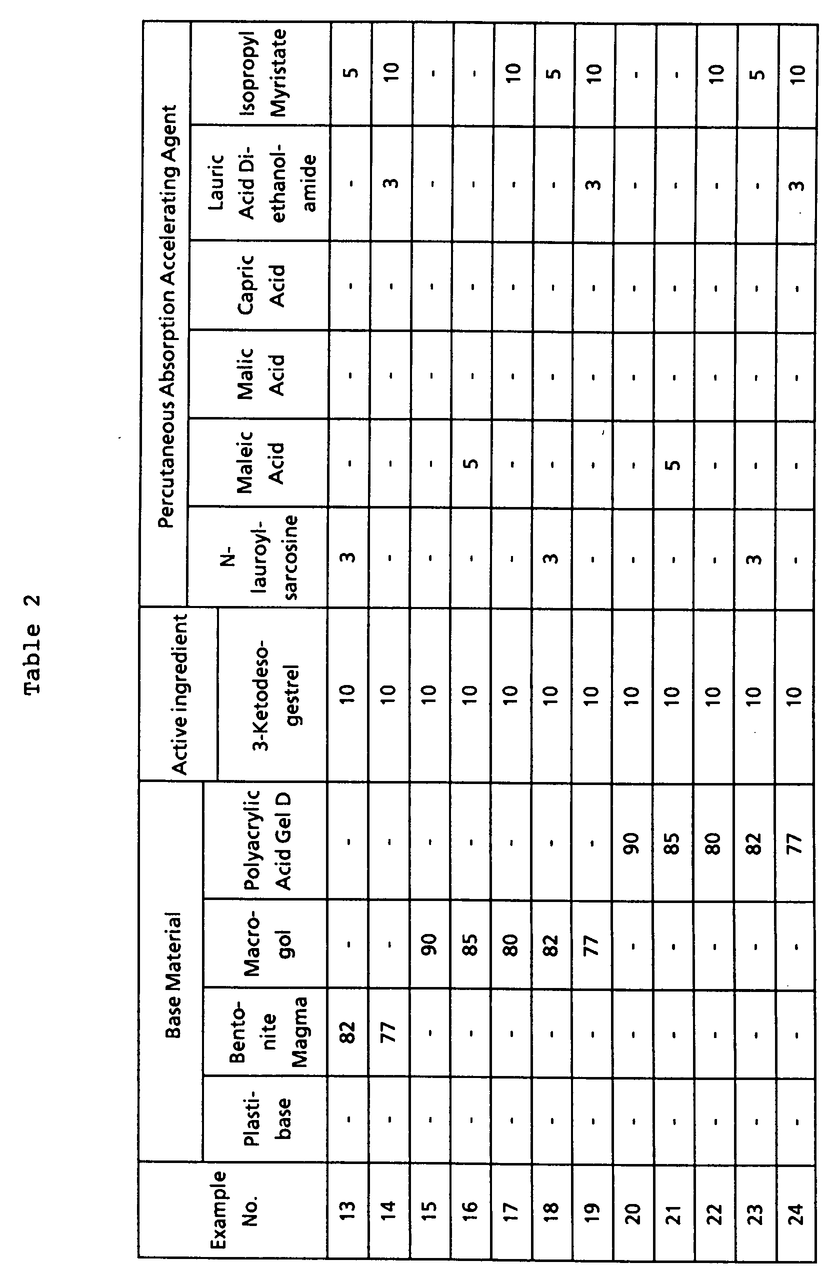

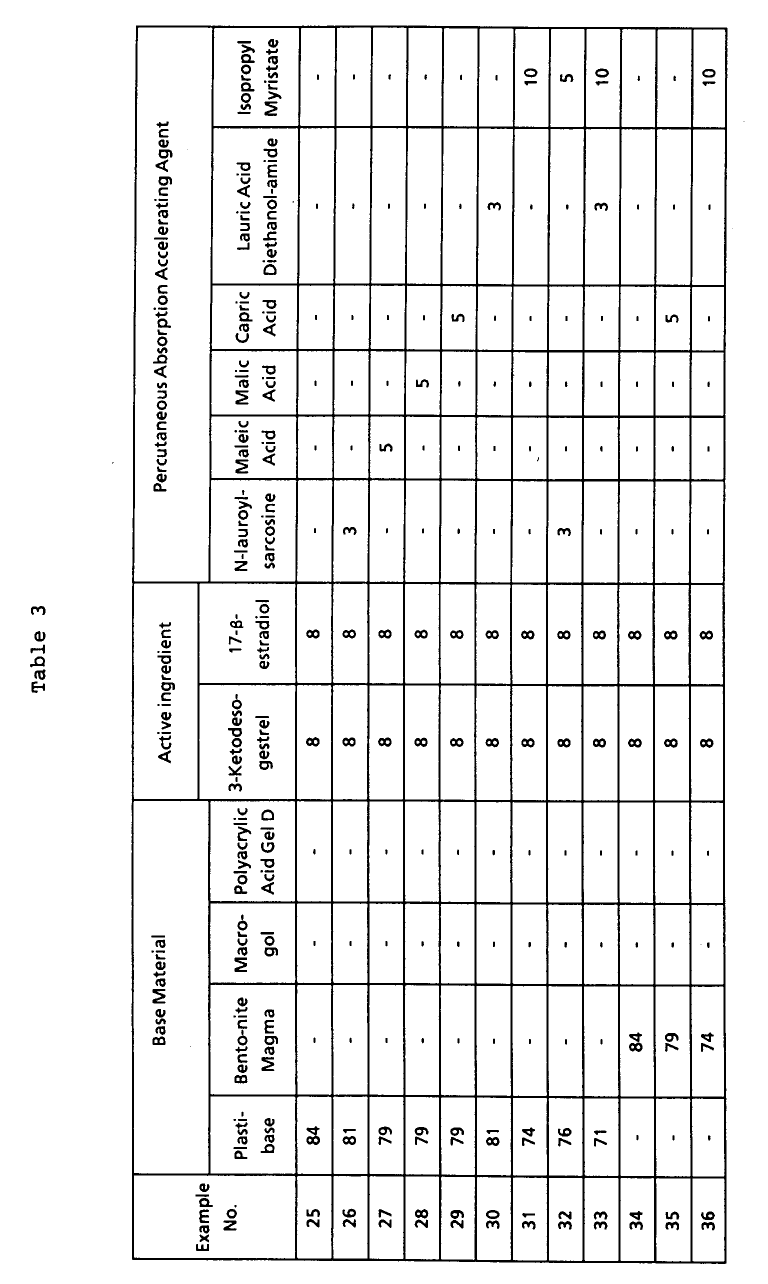

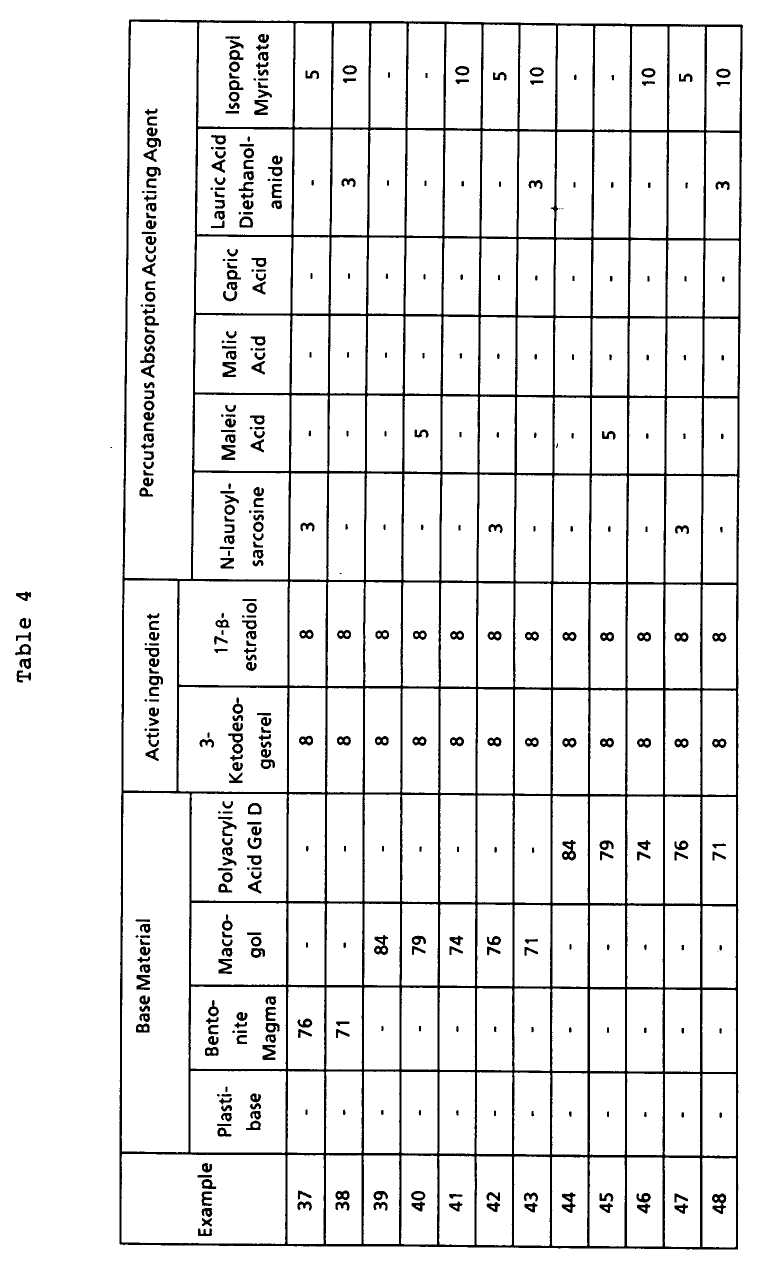

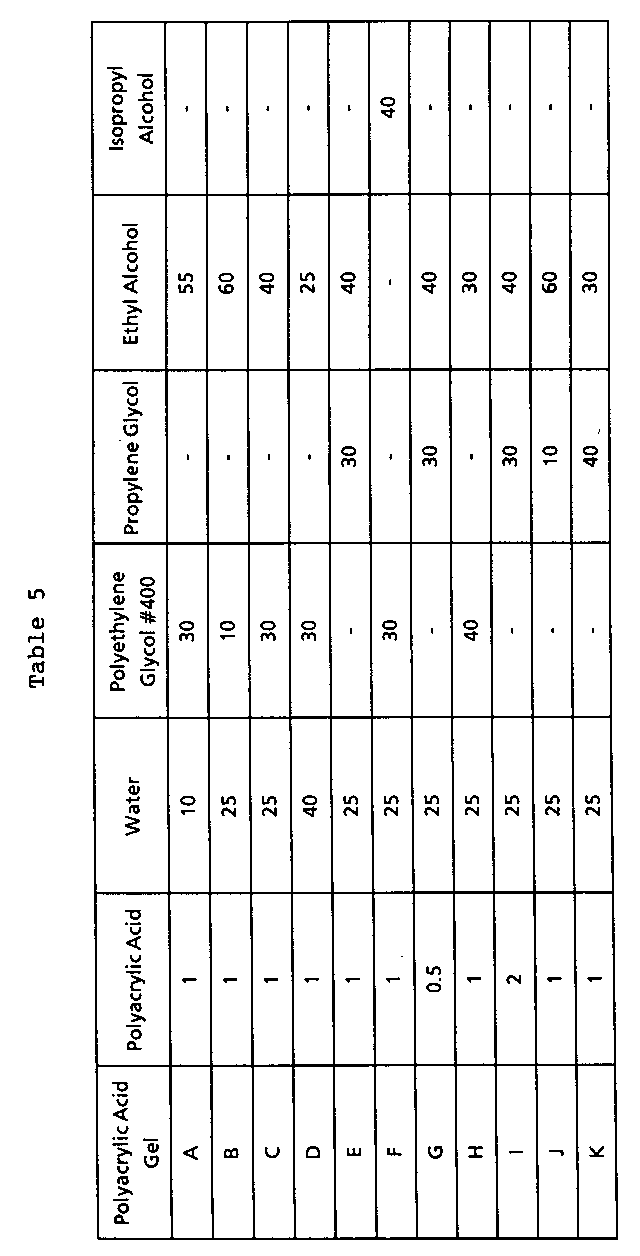

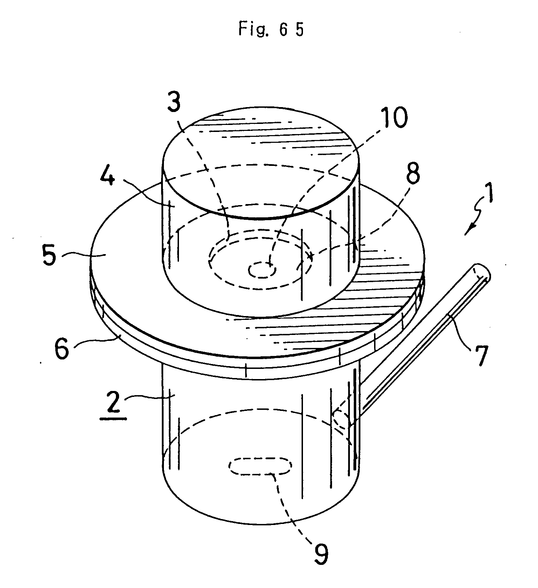

The preparation samples thus-obtained were subjected to a permeation test, using the diffusion cell 1 shown in Fig. 65. The diffusion cell 1 is composed of a bottomed cylindrical receptor vessel 2 and a bottomed cylindrical donor vessel 3 that has been placed above the vessel 2. The center of the bottom of the donor vessel 3 has an opening 4, and the bottom is fitted with a flange 5 extending outward and around it. The top of the receptor vessel 2 is fitted with a flange 6, and the wall thereof with a sampling mouth 7 projecting outwardly. The donor vessel 3 and the receptor vessel 2 have been combined hermetically and concentrically, with the flange 5 and the flange 6 facing each other. A magnet stirrer 9 is located in the receptor tank 2.

A hairless mouse (6 weeks of age, male) was sacreficed by dislocation of the cervical vertebrae, immediately the skin on the back was detached, the subcutaneous fat and the muscular tunics were removed, and thus skin specimens of approximately 5 cm x 5 cm in size were obtained. The thus-obtained skin specimen 8 was set in the diffusion cell 1 between the flange 5 and the flange 6, of the diffusion cell so that the opening 4 of the donor vessel 3 were completely sealed with the skin specimen 8.

200 mg of the percutaneous pharmaceutical preparation sample obtained above was uniformly coated over one surface of a circular (10 cm²) polyethylene terephthalate film 10, and the film 10 was attached to the center of the skin piece 8 with the coated preparation layer being kept in contact with the skin piece 8.

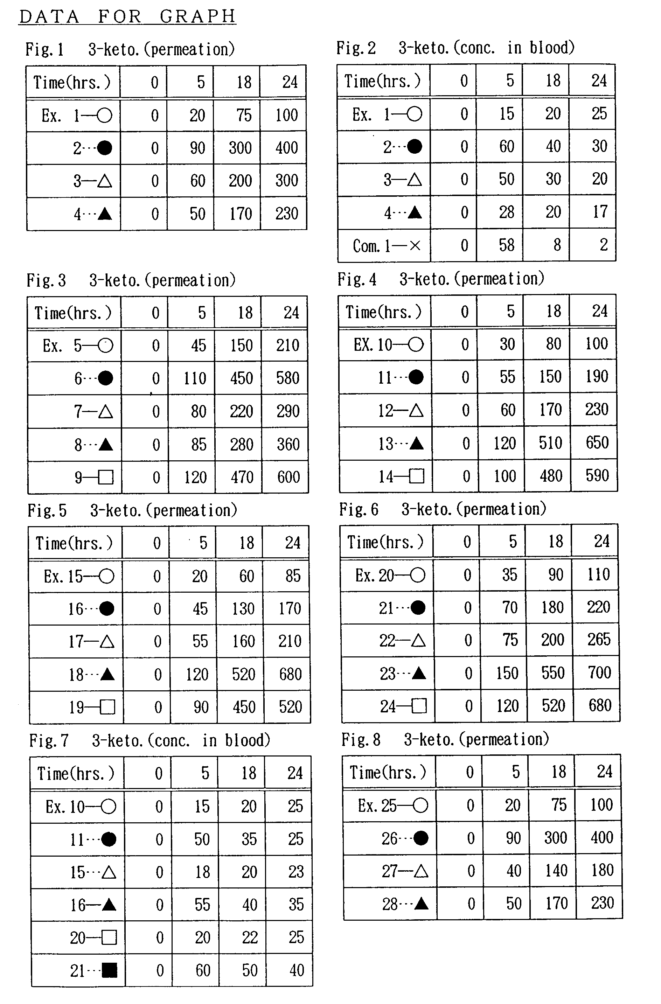

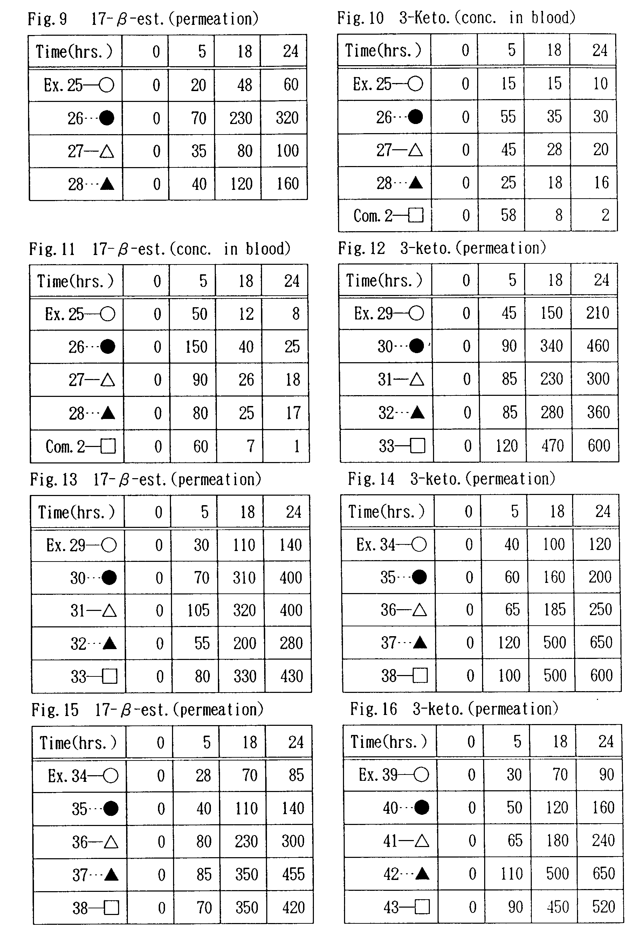

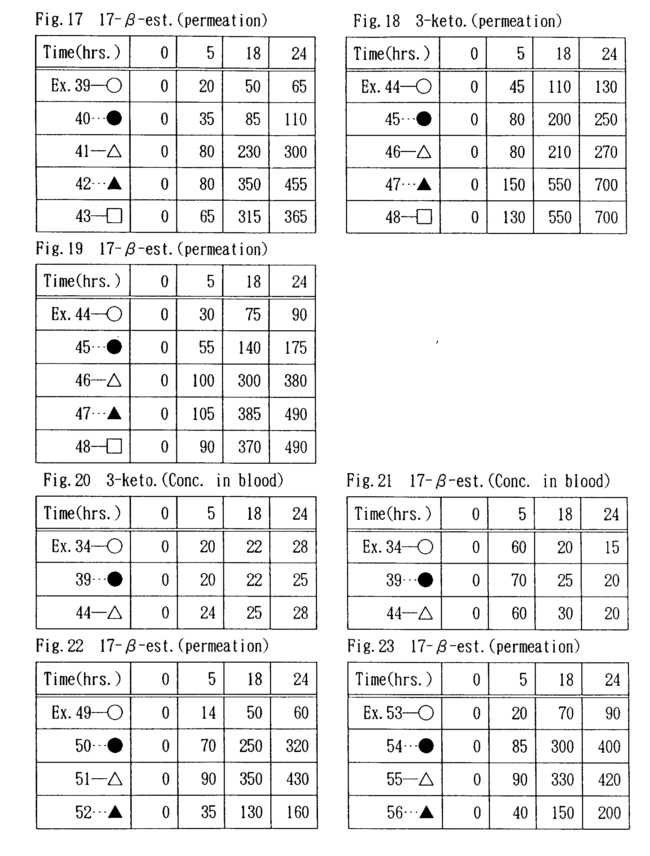

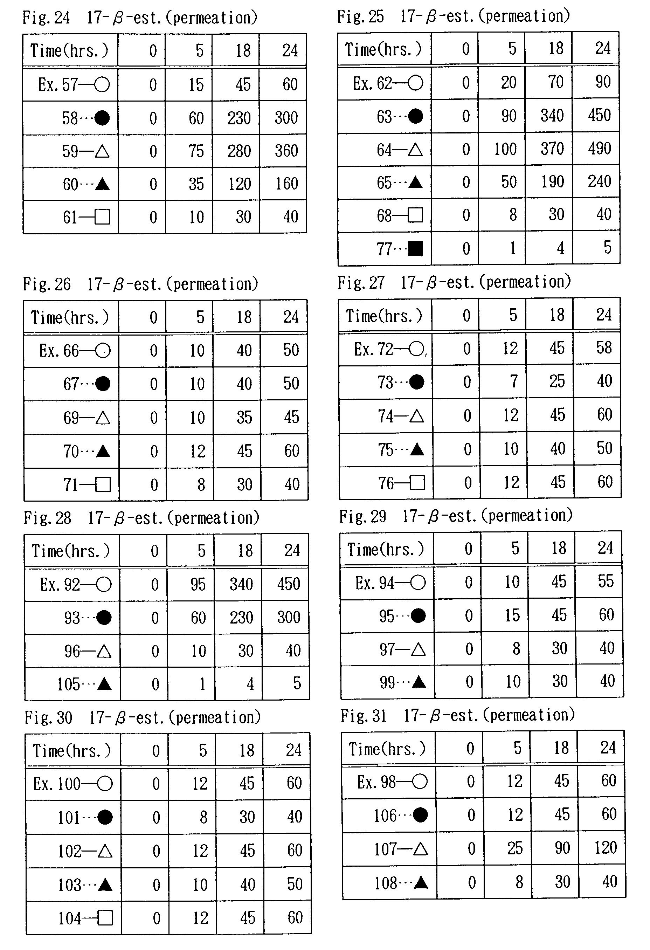

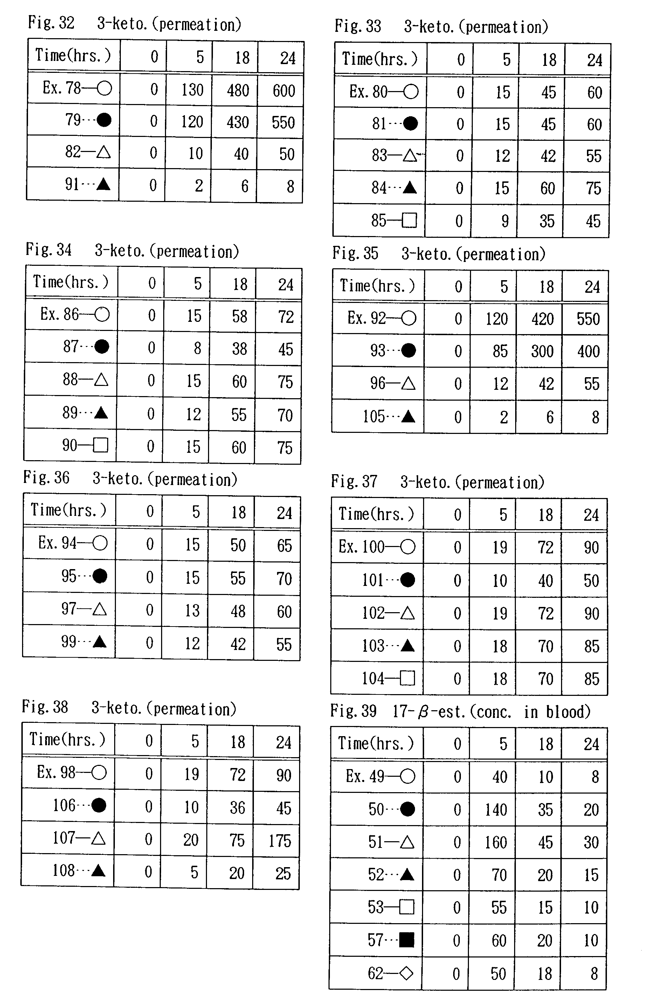

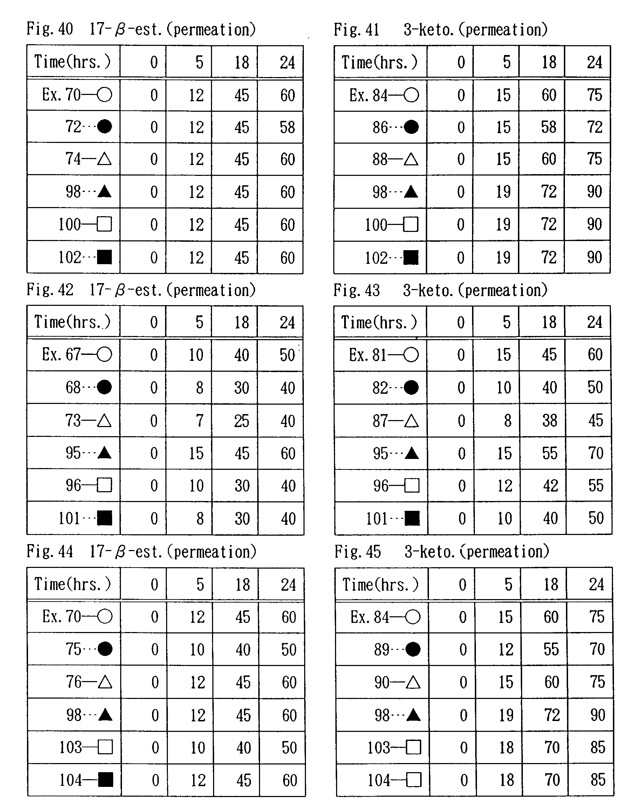

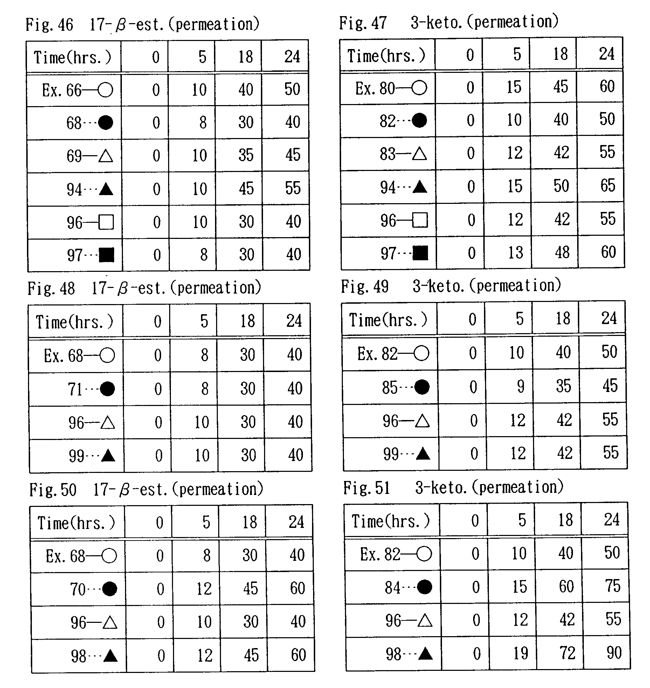

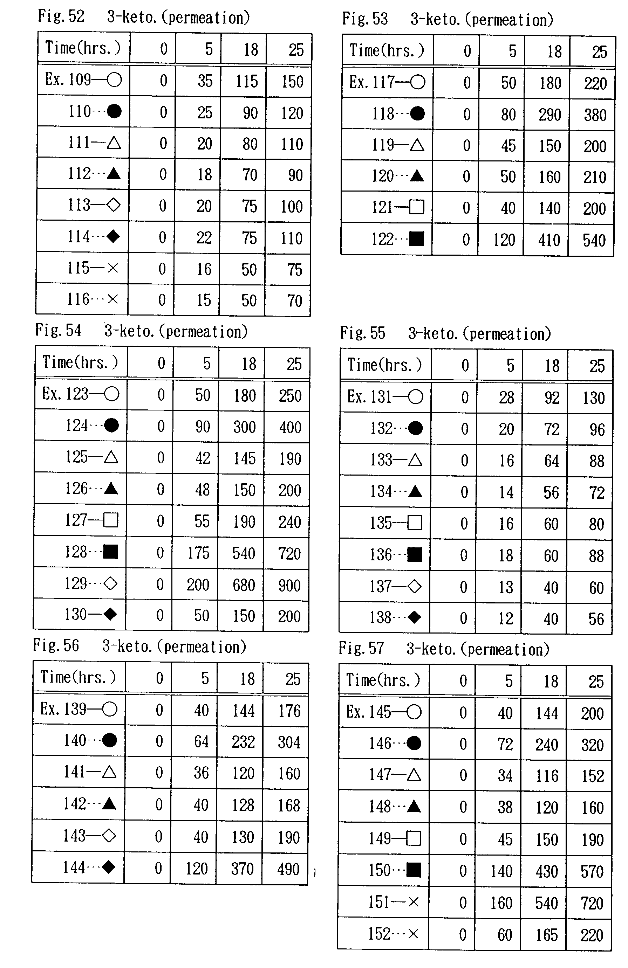

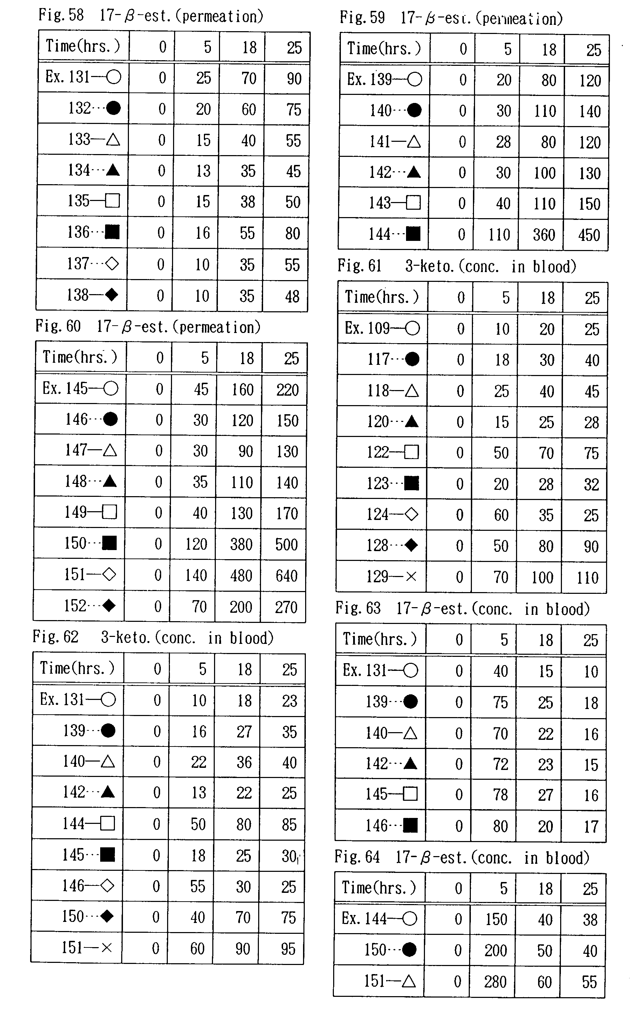

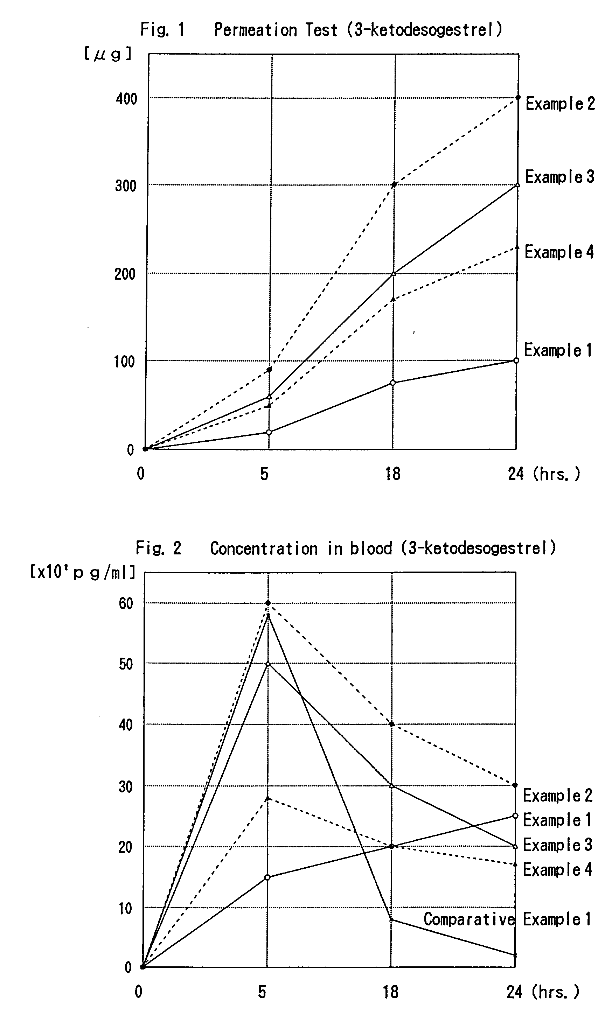

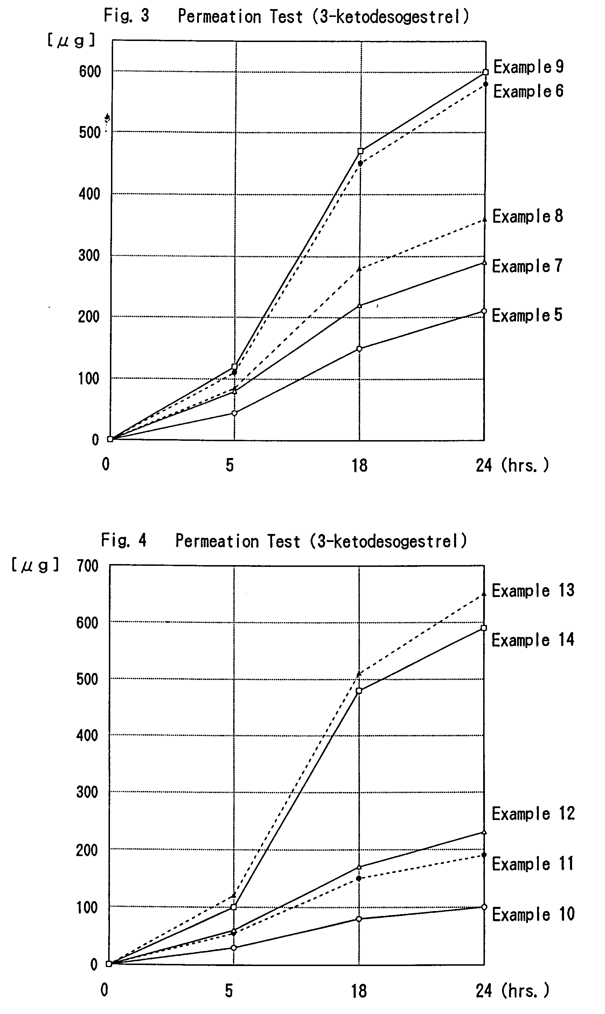

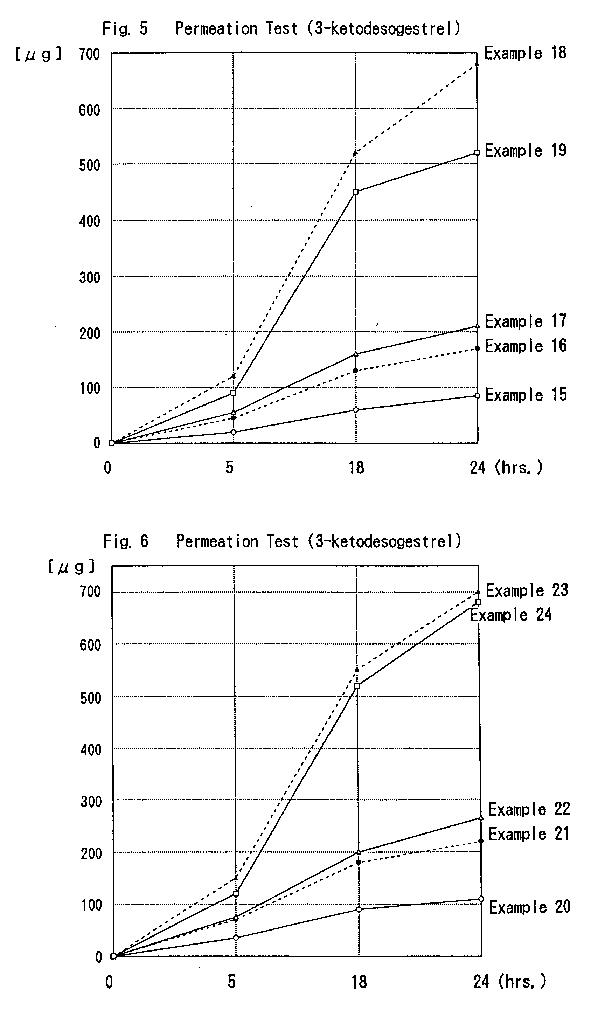

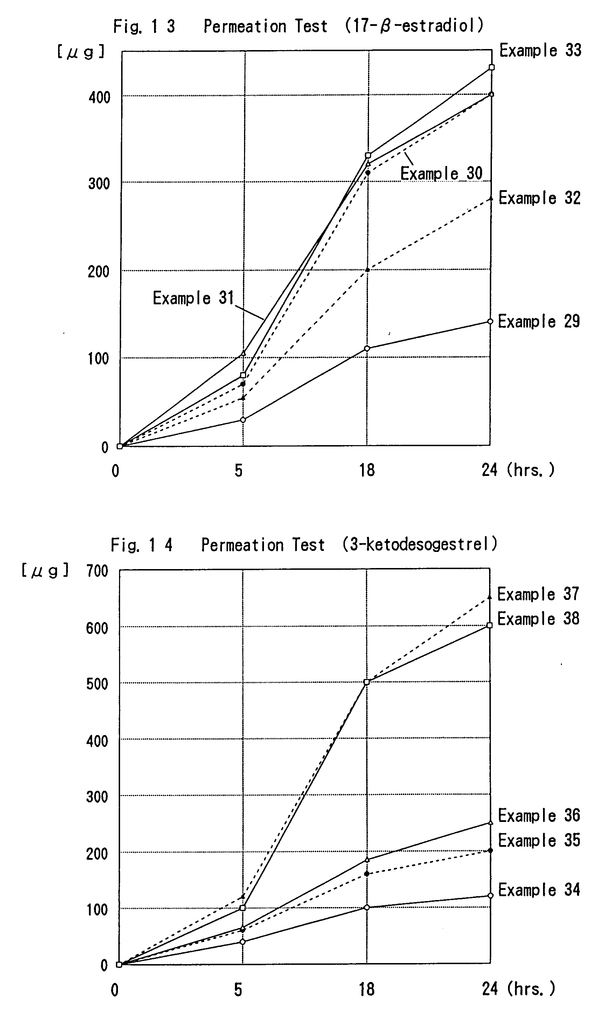

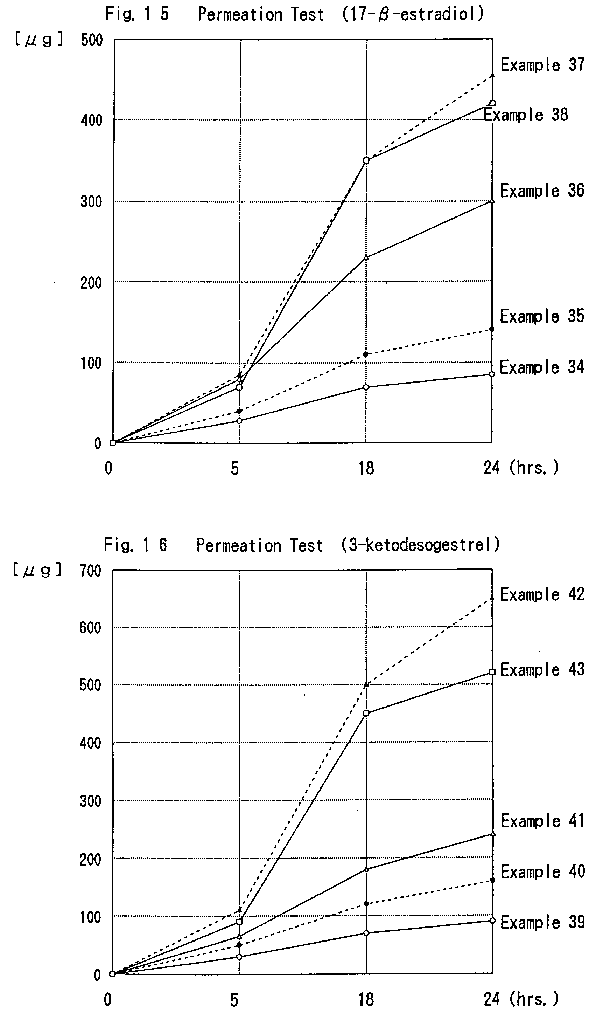

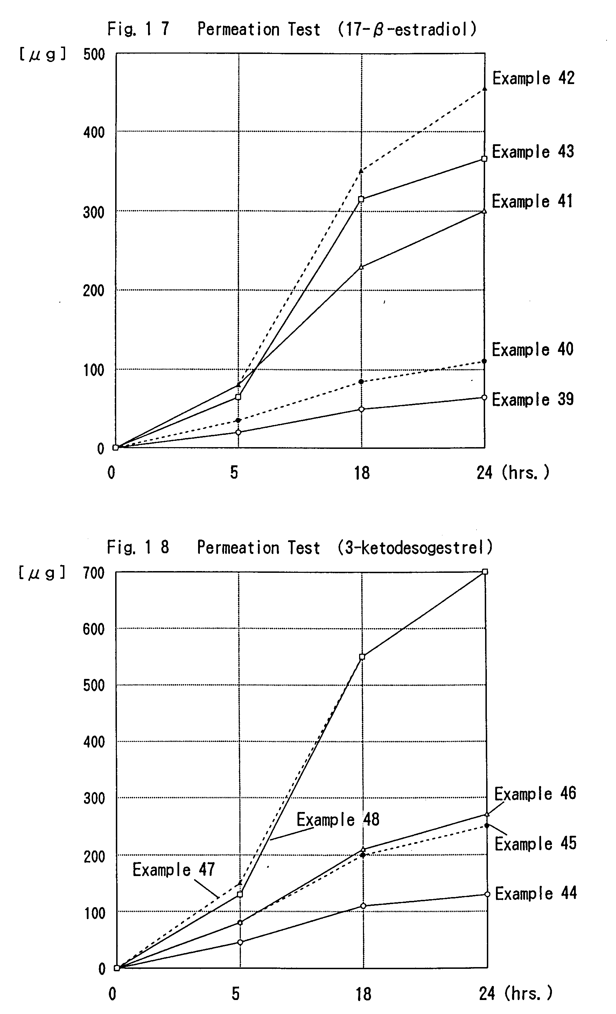

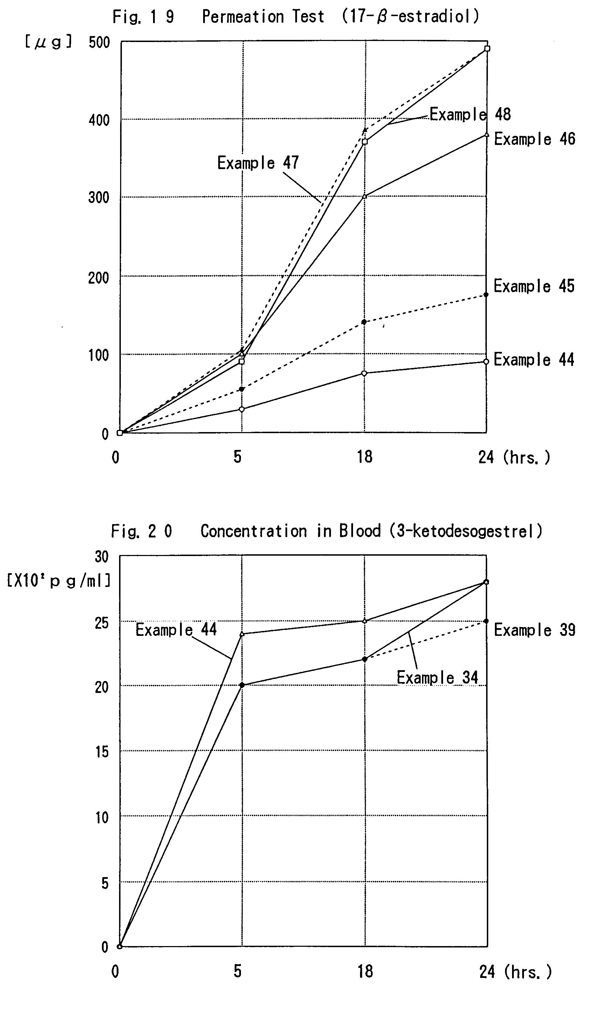

The receptor vessel 2 was filled with a receptor solution and set in a thermostatic device maintained at 37°C, while the magnet stirrer was rotated with a magnet stirring device to agitate the solution. 5, 18 and 24 hours after the start of the test, one ml of the receptor solution was sampled out from the sampling duct 7 and the amount(s) of the active ingredient(s) in the sampled receptor solution was/were measured by high-performance liquid chromatography. Every time the receptor solution was sampled, one mil of fresh receptor solution was provided. This test was performed for 3 specimens of each type of preparation, and the average of the 3 values so obtained was caluculated. The results are shown in Figs. 1, 3 to 6, 8, 9 and 12 to 19.

The receptor solution was obtained as follows;

A buffer solution consisting of 5 x 10⁻⁴ M NaH₂PO₄, 2 x 10⁻⁴ M Na₂HPO₄, 1.5 x 10⁻¹ M NaCl and 10 ppm of gentamicin in distilled water was adjusted to pH 7.2 with an aqueous sodium hydroxide solution, and the receptor solution was obtained by addition of polyethylene glycol 400 so that the concentration of the polyethylene glycol should be 20% by weight.

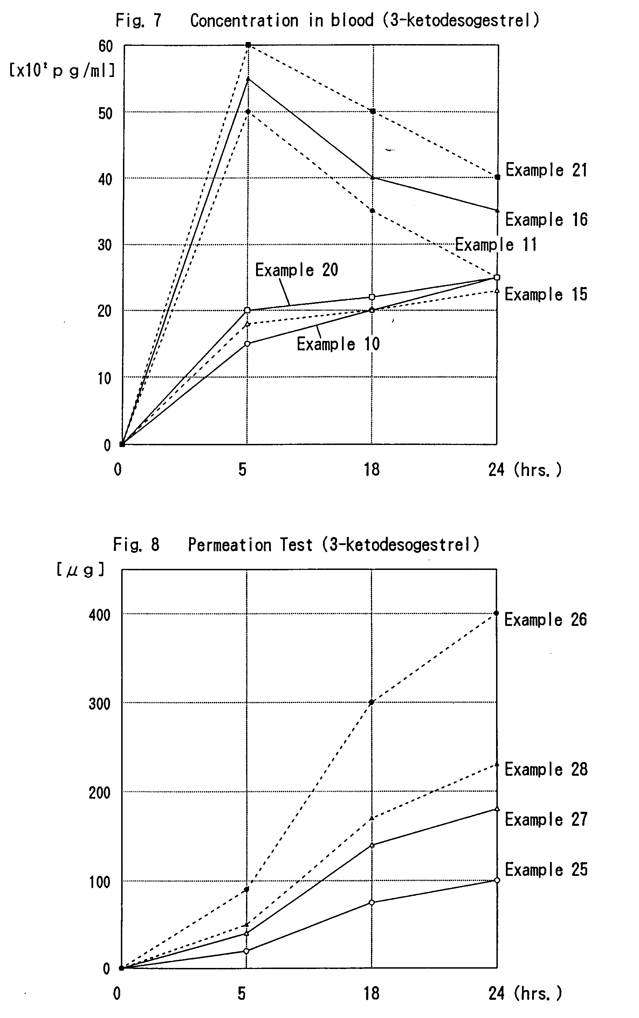

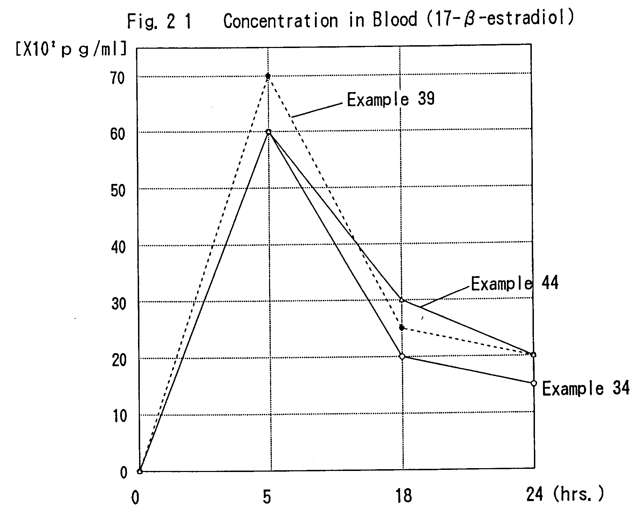

The preparation sample obtained in each of Examples 1 to 4, 10, 11, 15, 16, 20, 21, 25 to 28, 34, 39 and 44 was applied to rats in order to measure the concentrations of the active ingredients in the blood. Precisely, the whole back of a Wister rat (7 weeks of age, male) was completely shaven, and it was confirmed to be neither wounded nor inflamed. Twenty-four hours after the shaving, a one inch-square polyethylene terephthalate film, one surface of which had been coated with 200 mg of the preparation sample, was applied to the balded back. Five, eighteen and twenty-four hours after the application, blood was drawn. The concentration(s) of the active ingredient(s) in the thus-drawn blood was/were measured by radio-immunoassay. The results are shown in Figs. 2, 7, 10, 11, 20 and 21.

### Comparative Example 1:

100 mg of 3-ketodesogestrel were dissolved in 100 ml of an aqueous solution prepared by dissolving 0.01 part by weight of carboxymethyl cellulose in 100 parts by weight of water, to obtain a pharmaceutical solution. One ml of the thus-obtained pharmaceutical solution was placed into the stomach of a Wister rat (7 weeks of age, male) using a glass syringe and a cannula, and the concentration of the 3-ketodesogestrel in the blood of the rat was measured in the same manner as in Example 1. The results are shown in Fig. 2.

### Comparative Example 2:

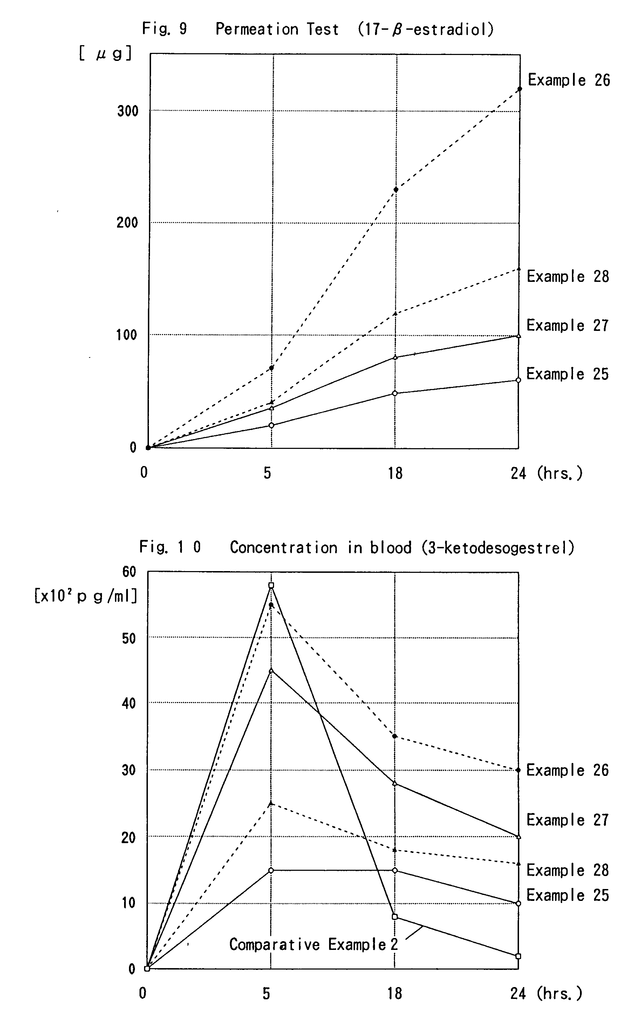

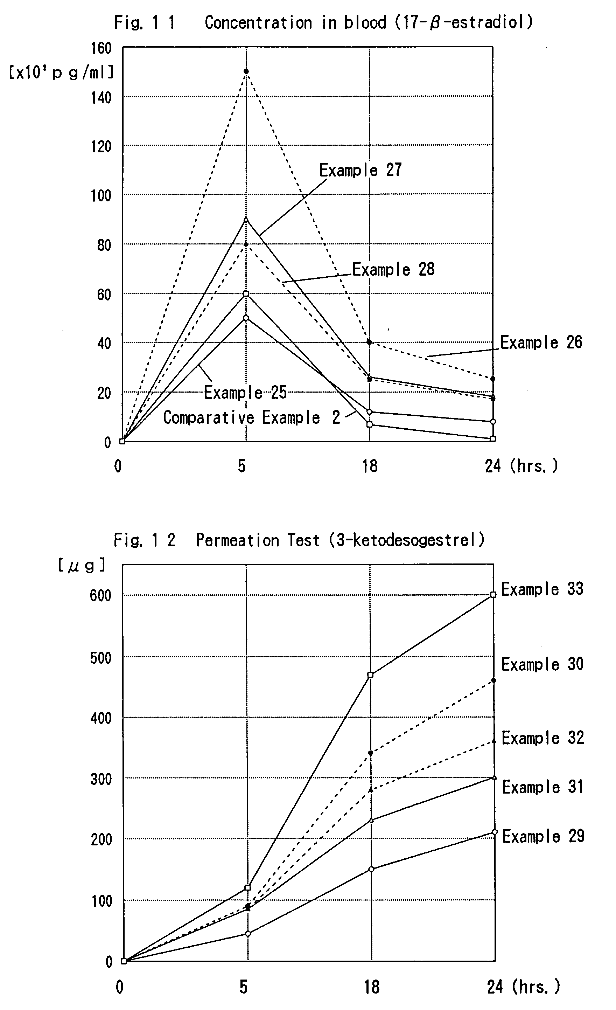

80 mg of 3-ketodesogestrel and 80 mg of 17-β-estradiol were dissolved in 100 ml of an aqueous solution prepared by dissolving 0.01 part by weight of carboxymethyl cellulose in 100 parts by weight of water, to obtain a pharmaceutical solution. One ml of the thus-obtained pharmaceutical solution was placed into the stomach of a Wister rat (7 weeks of age, male) with a glass syringe and a cannula, and the concentrations of the active ingredients in the blood of the rat were measured in the same manner as in Example 1. The results are shown in Figs. 10 and 11.

### Examples 49 to 108:

In the same manner as in Example 1, various percutaneous pharmaceutical preparation samples were obtained from pre-determined amounts (parts by weight) of the base material, the active ingredient(s) and the percutaneous absorption accelerating agent(s) shown in Tables 6 to 11.

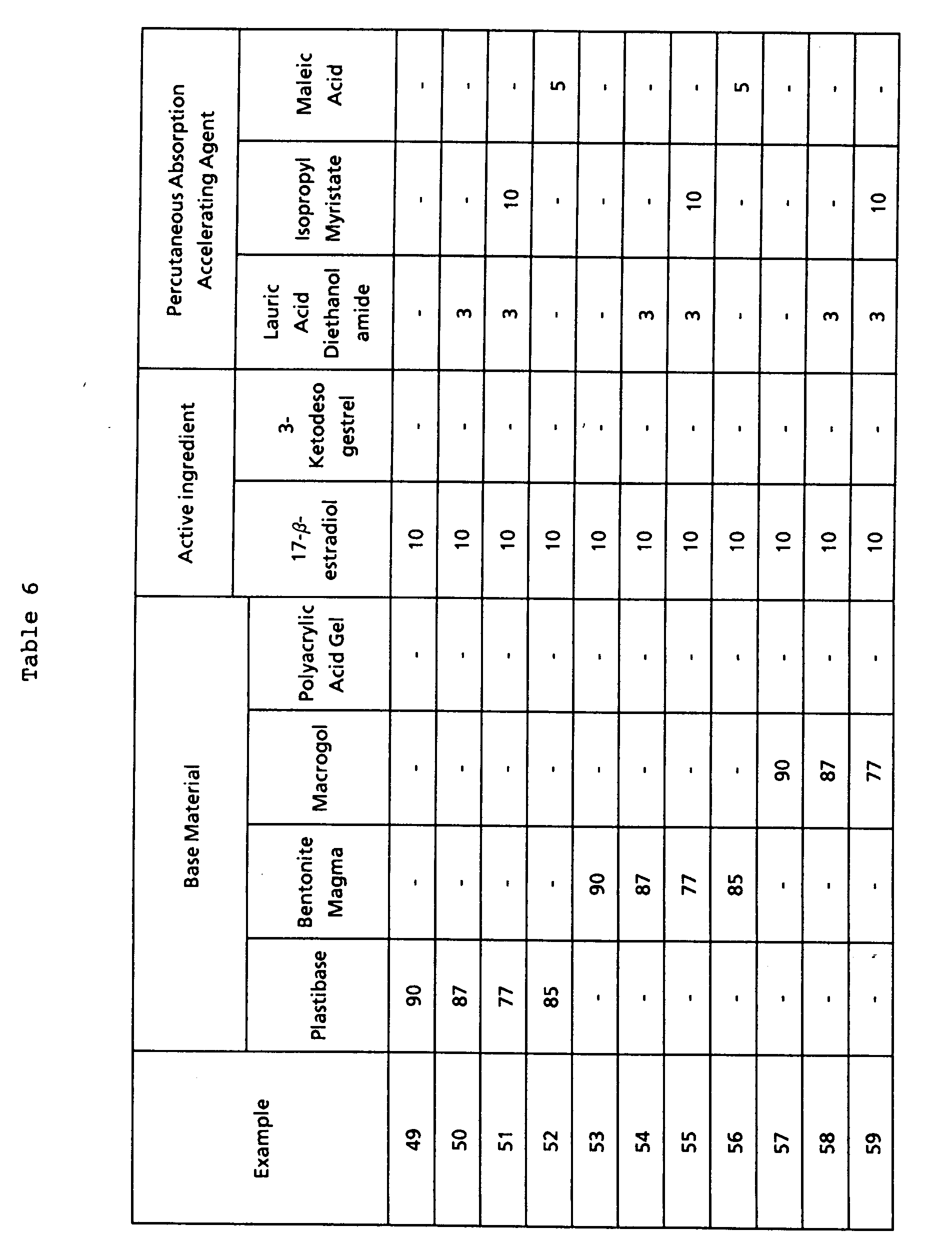

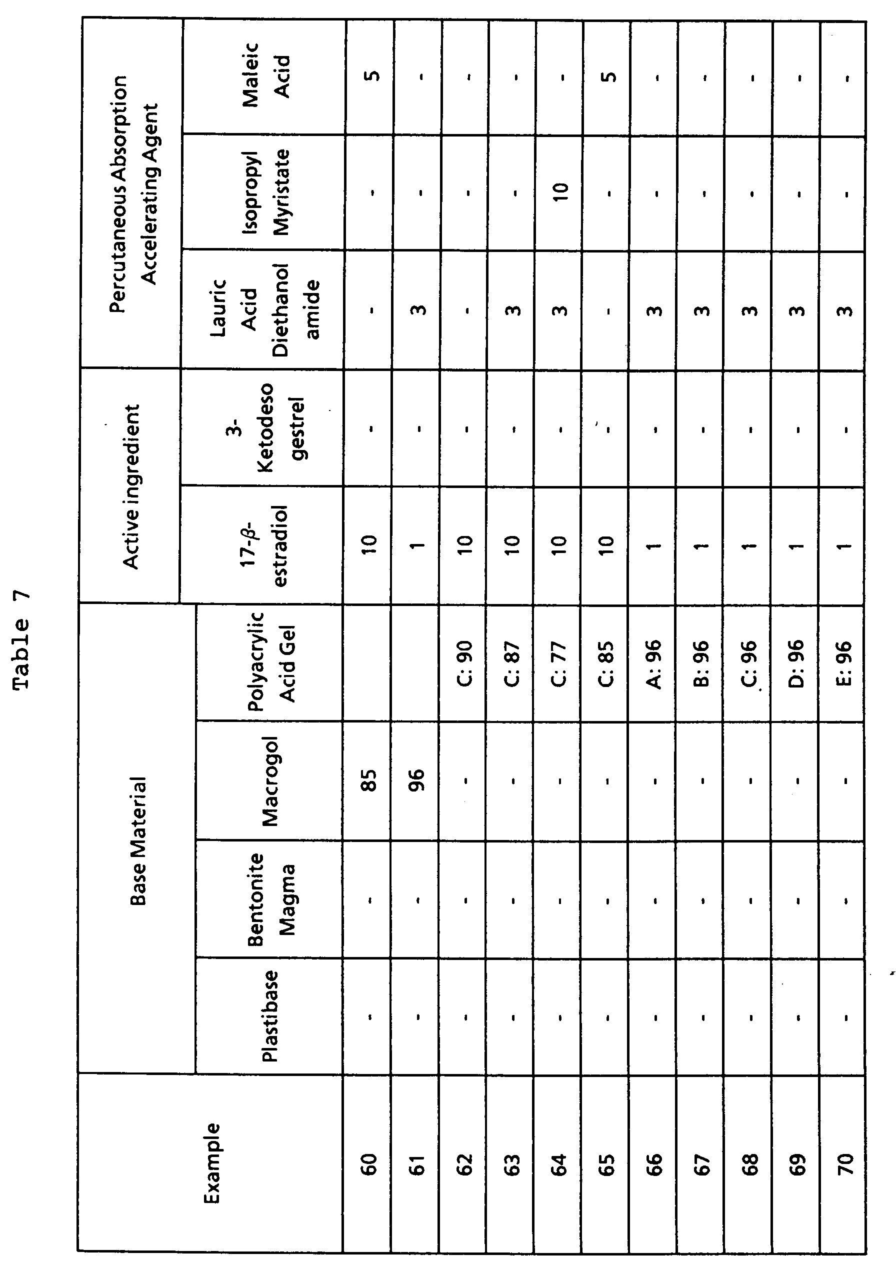

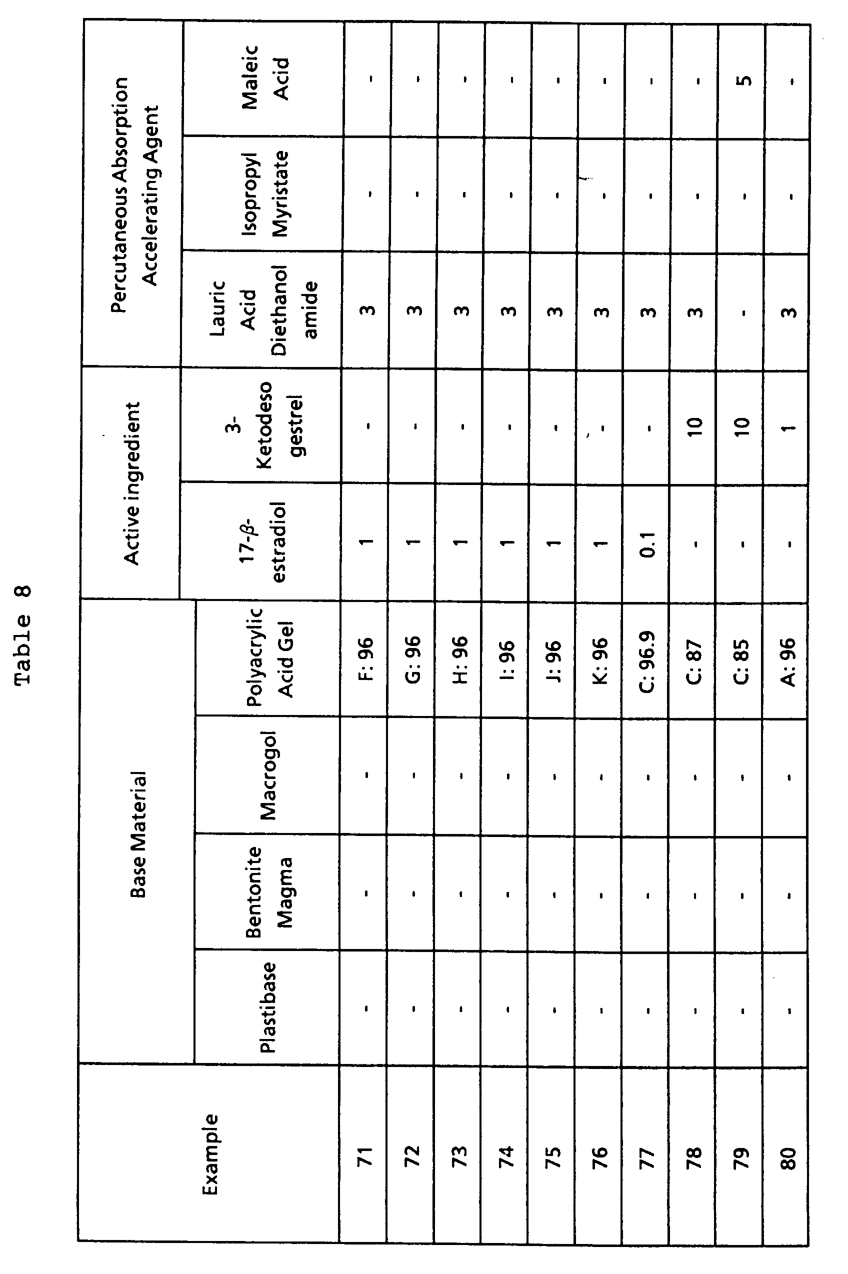

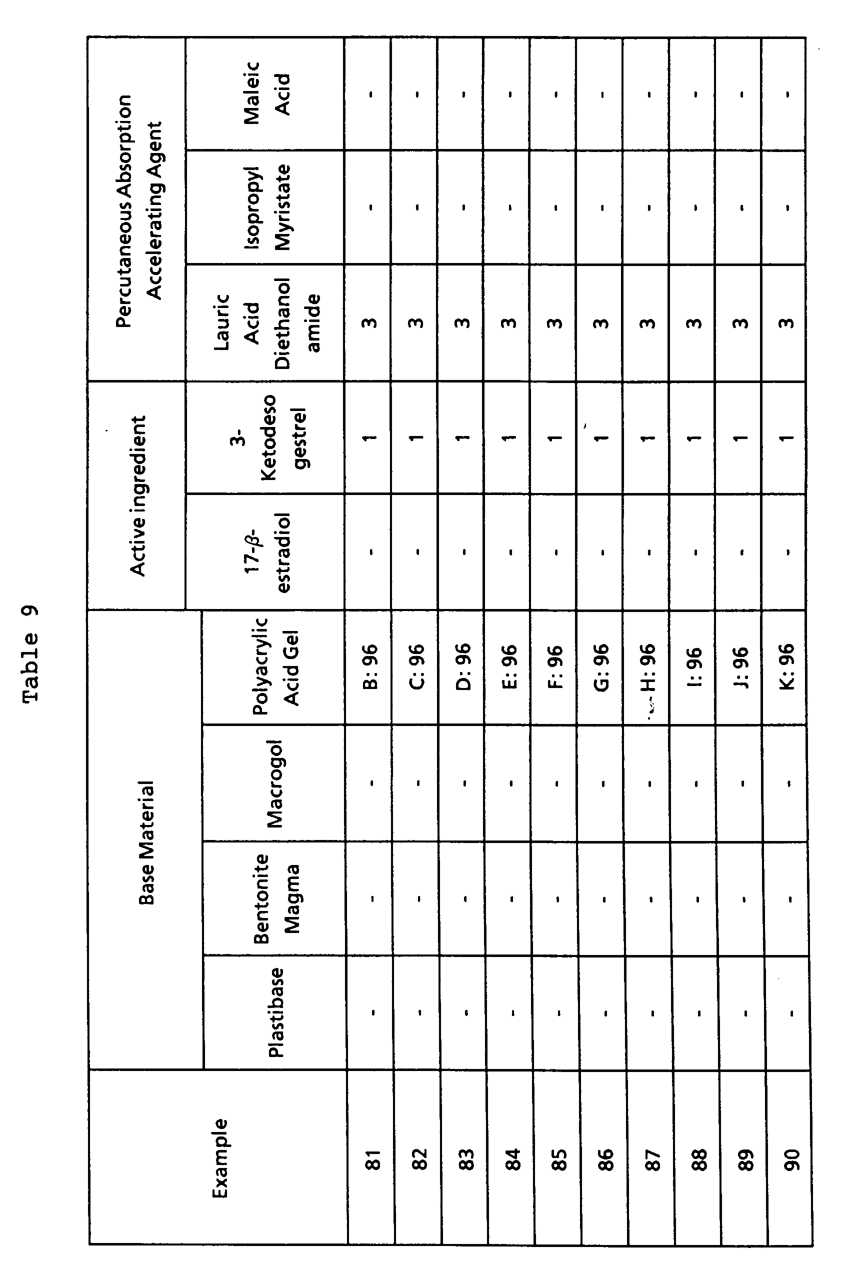

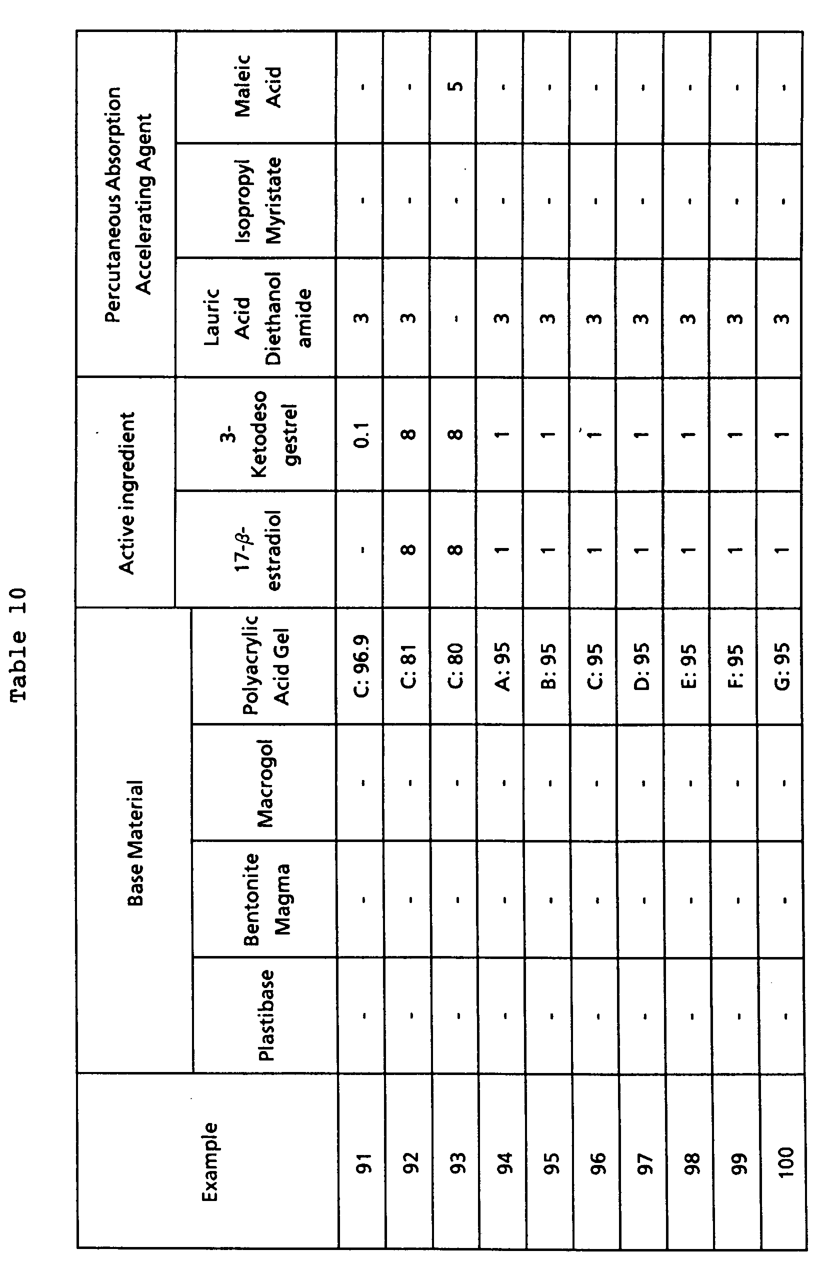

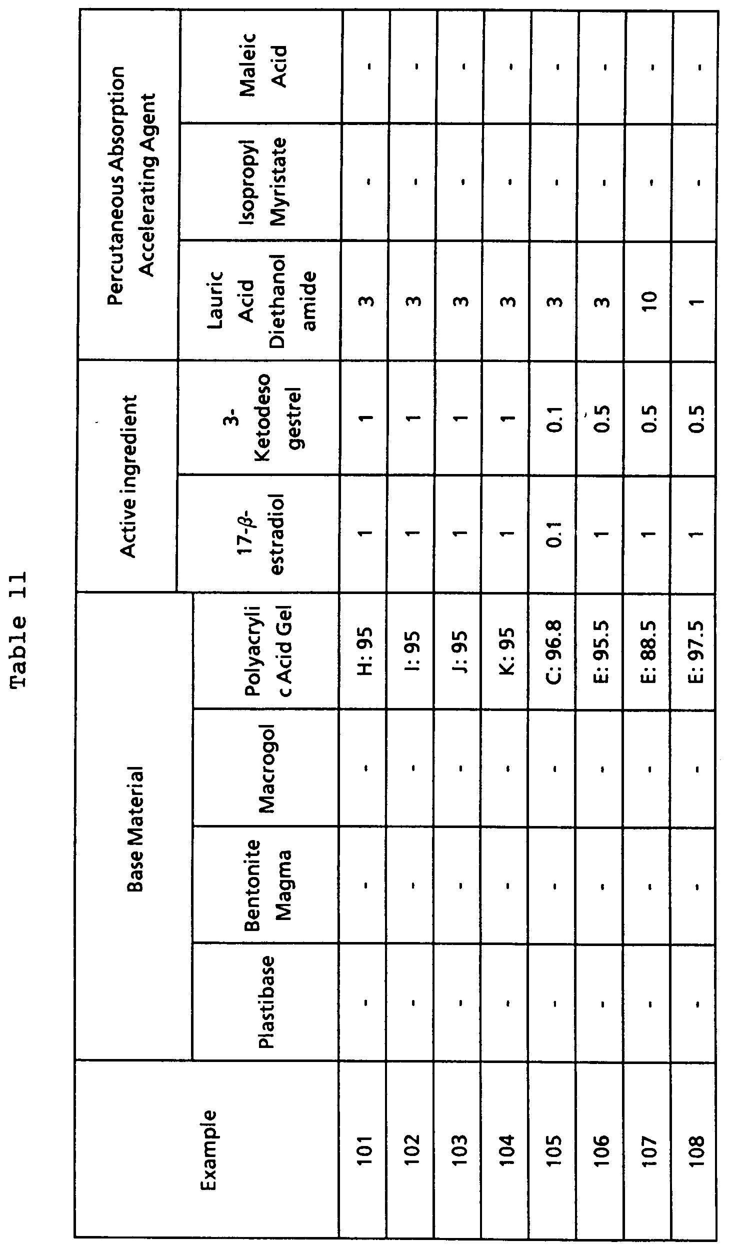

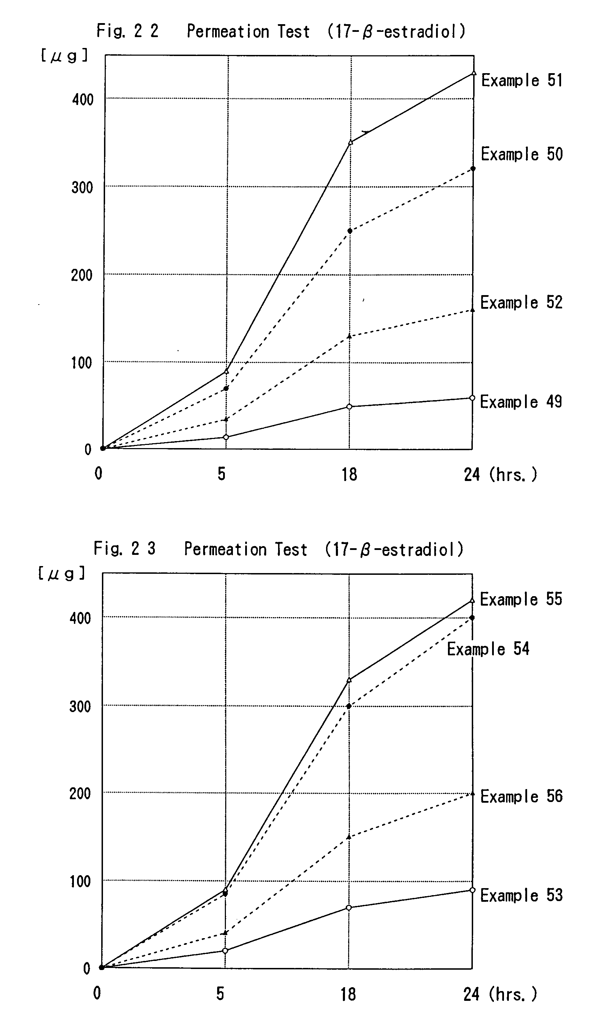

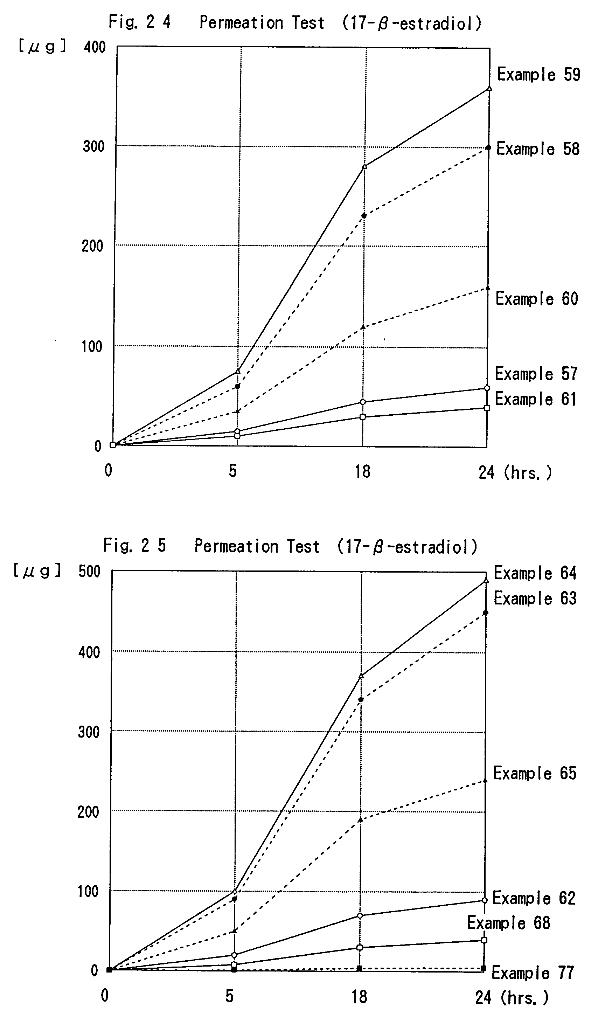

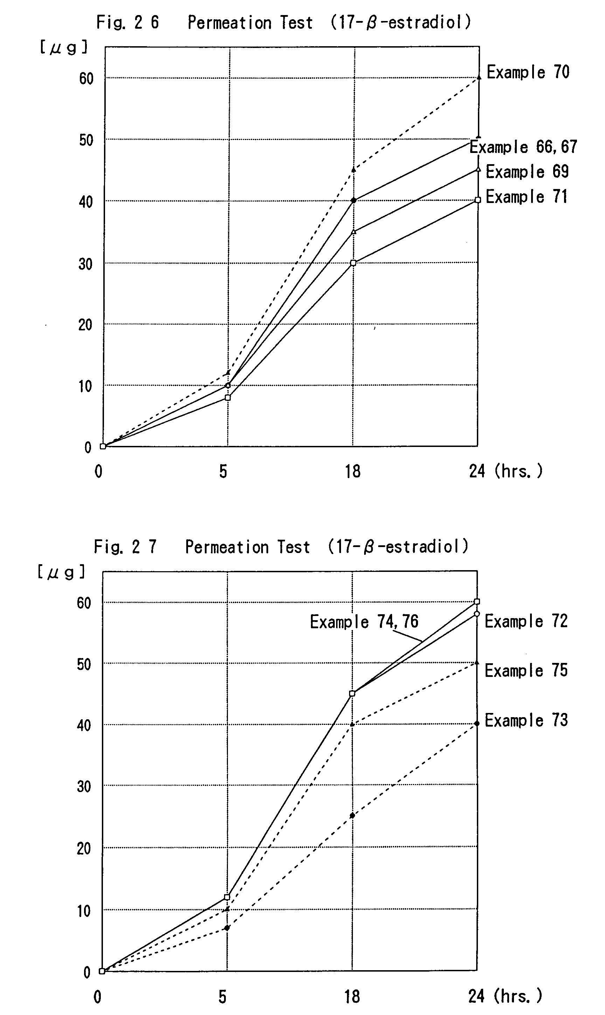

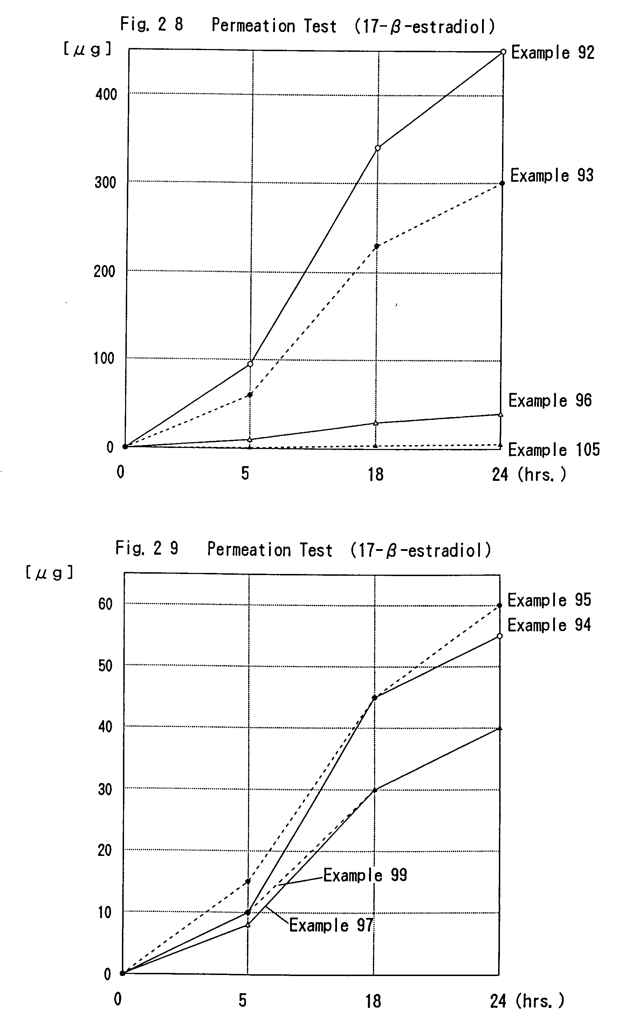

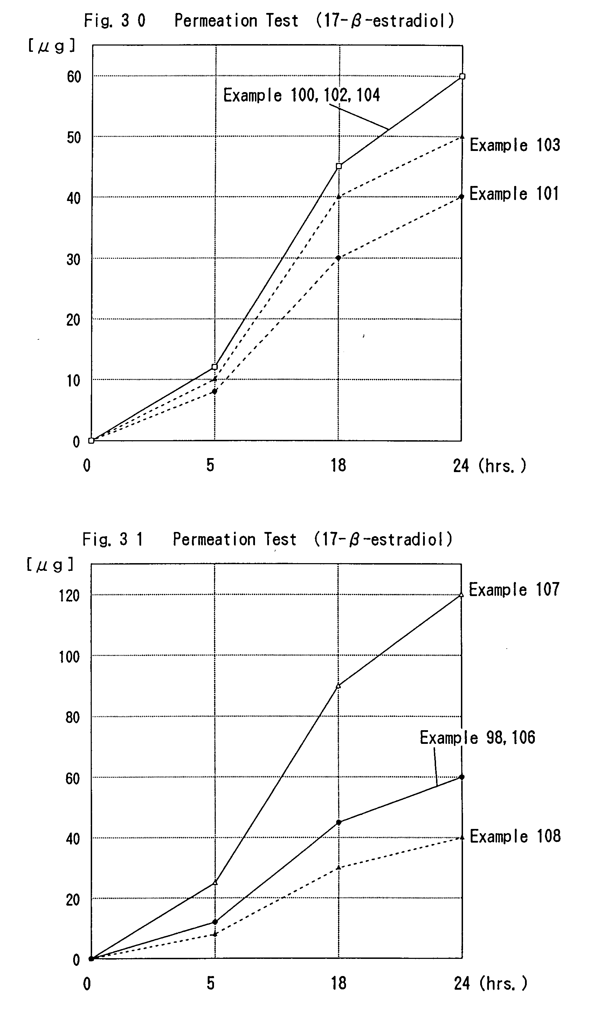

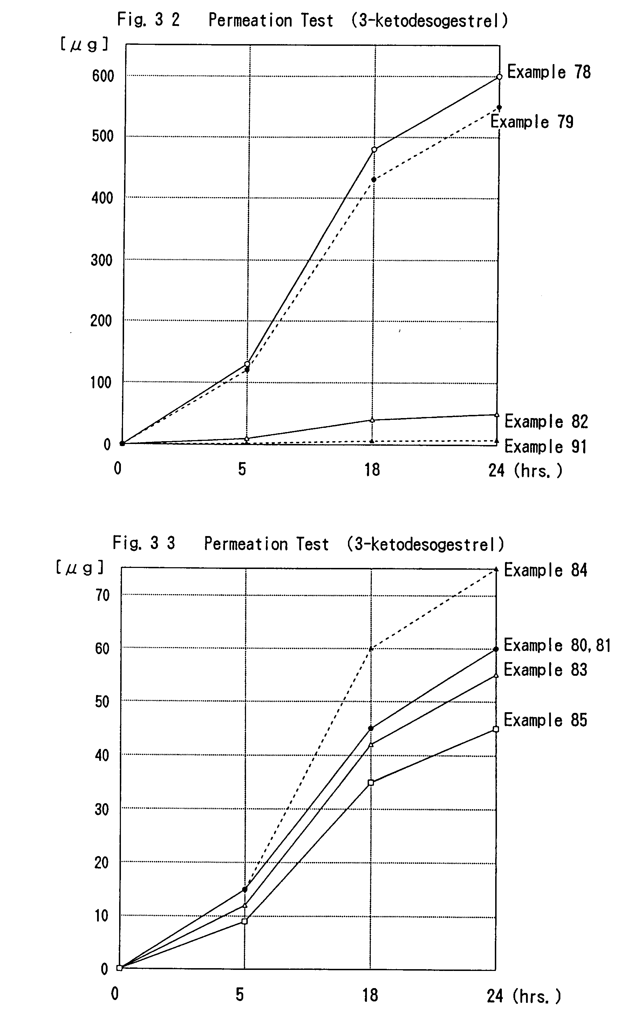

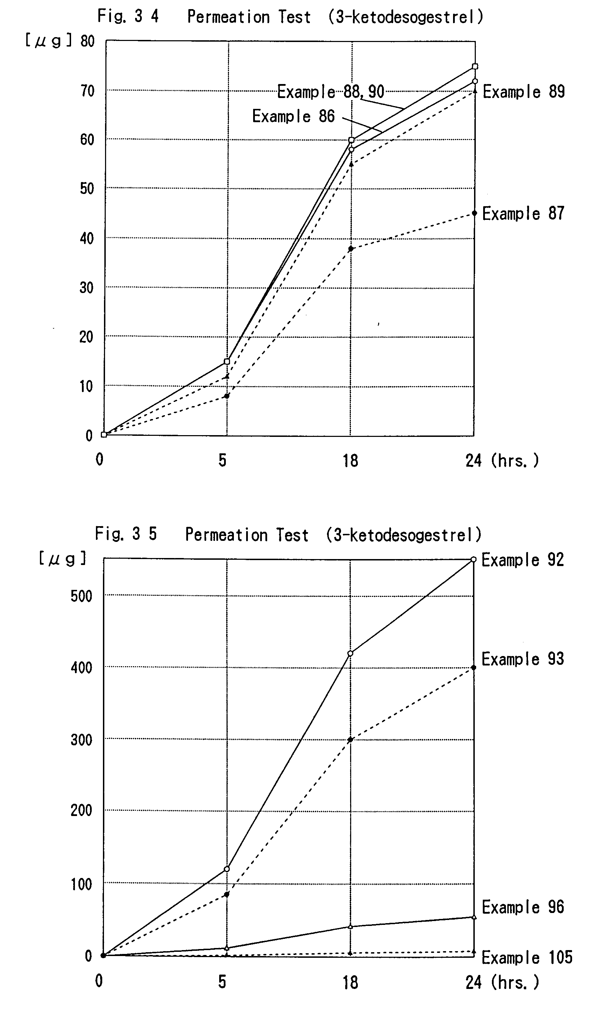

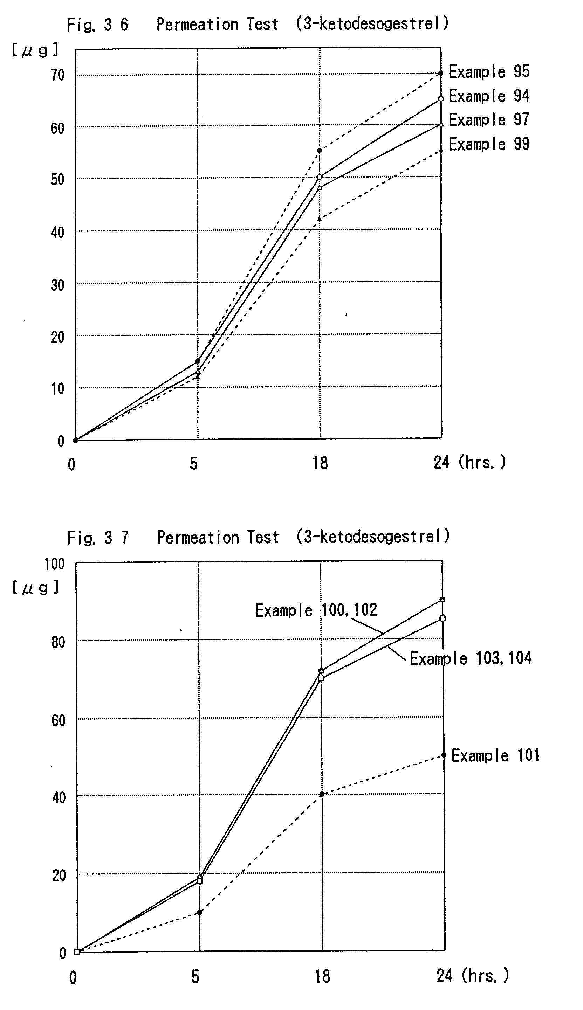

The preparation samples thus-obtained were subjected to the same permeation test as that in Example 1, using the diffusion cell 1 shown in Fig. 65. The test results are shown in Figs. 22 to 38.

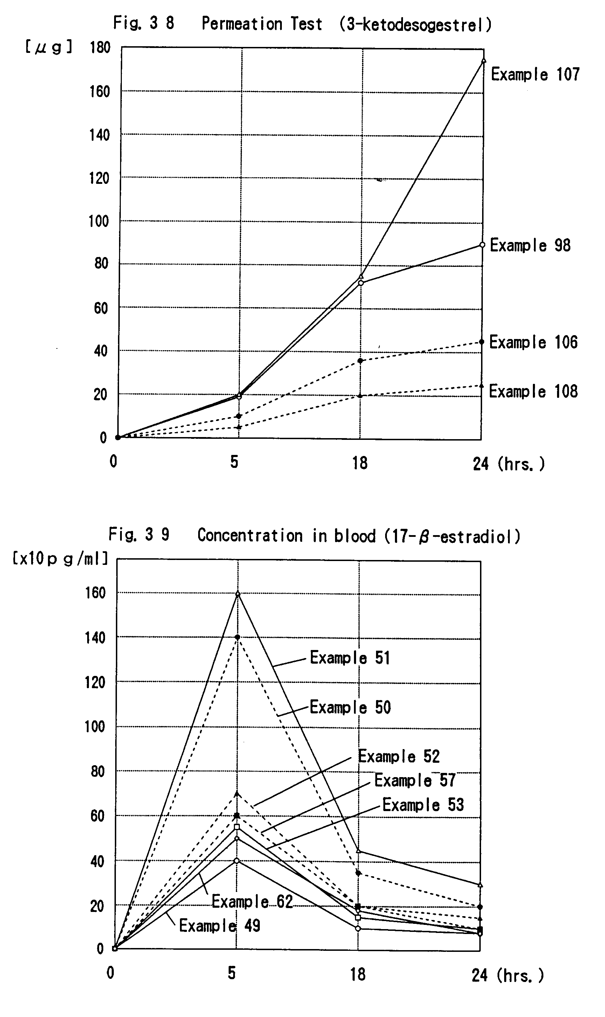

The preparation samples obtained in Examples 49 to 53, 57 and 62 were tested also in the same manner as in Example 1 in order to measure the concentration of the active ingredient in the blood. The results are shown in Fig. 39.

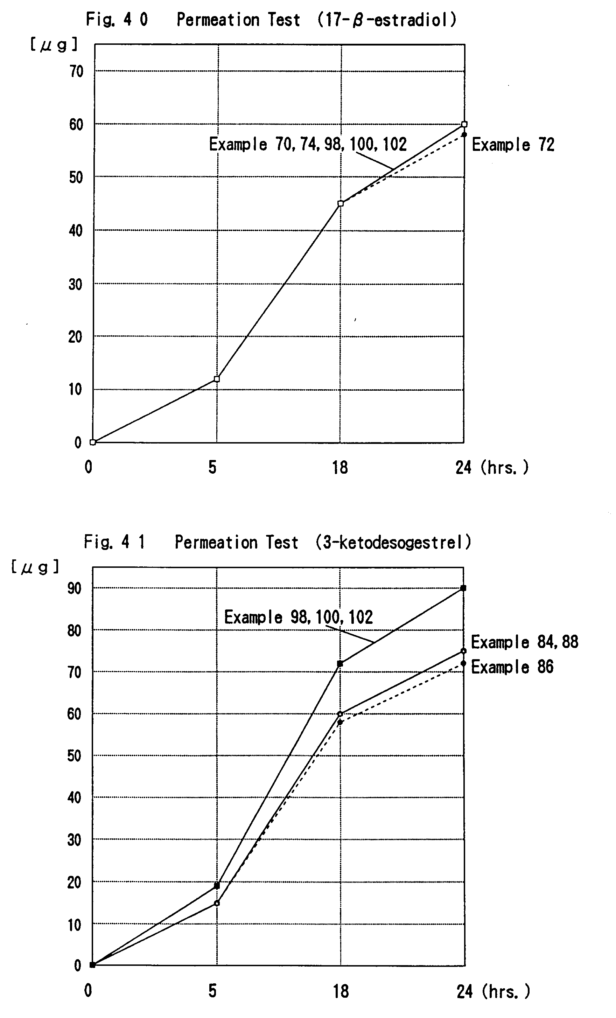

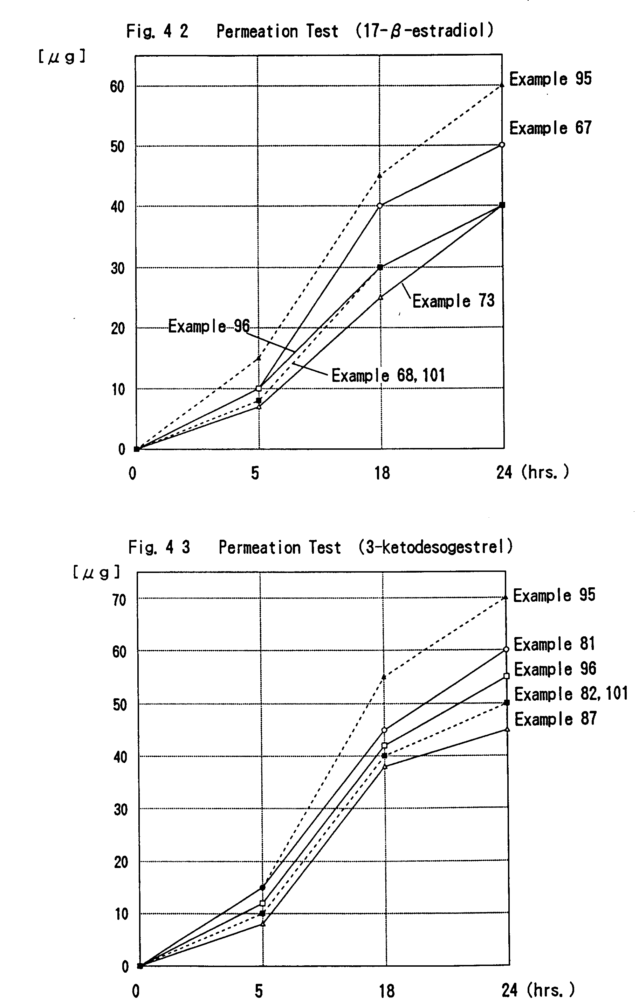

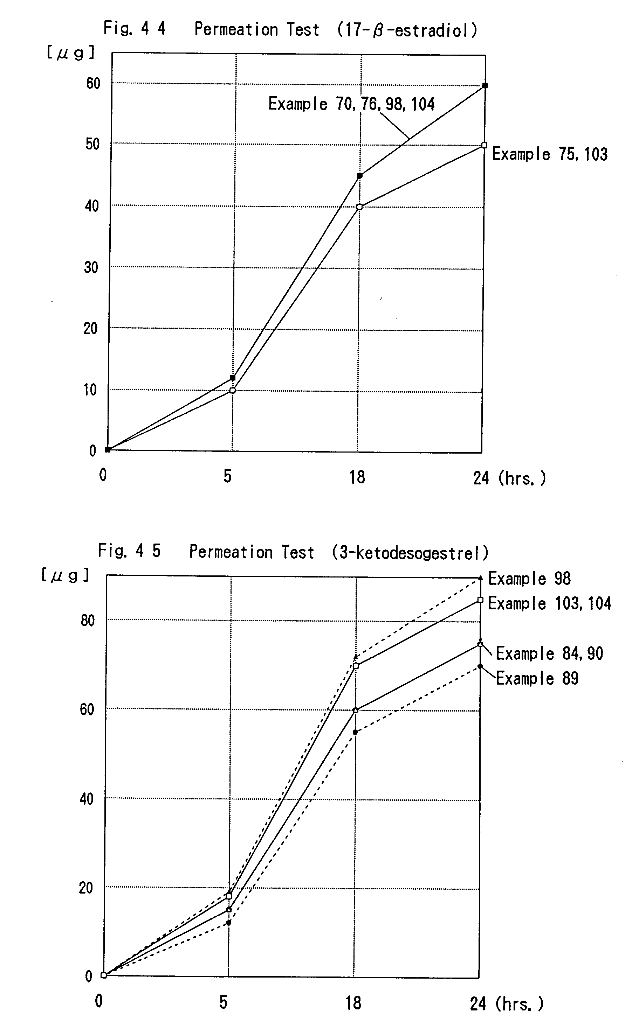

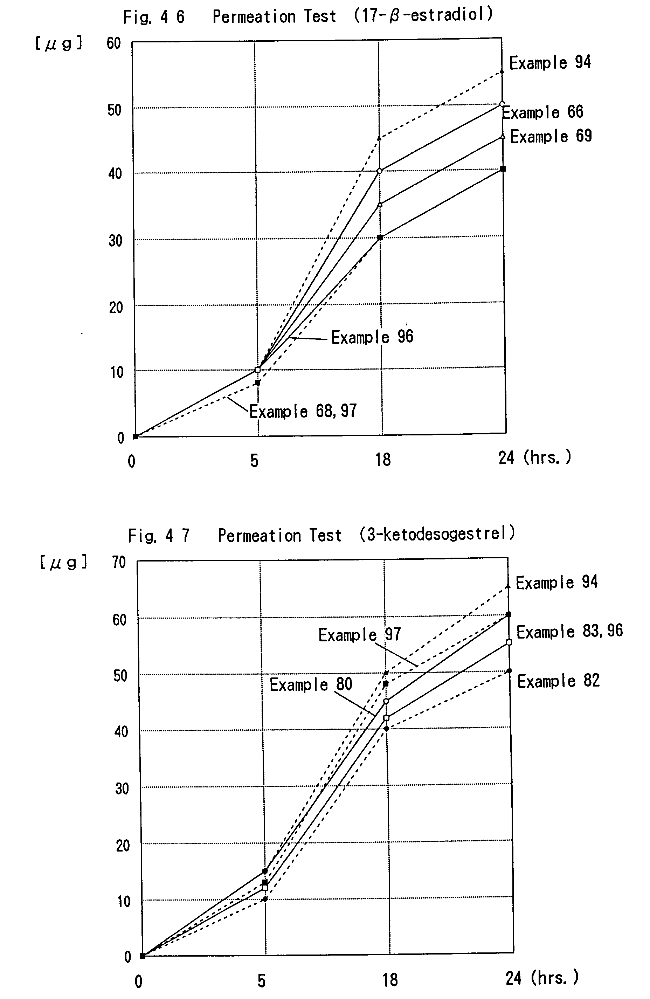

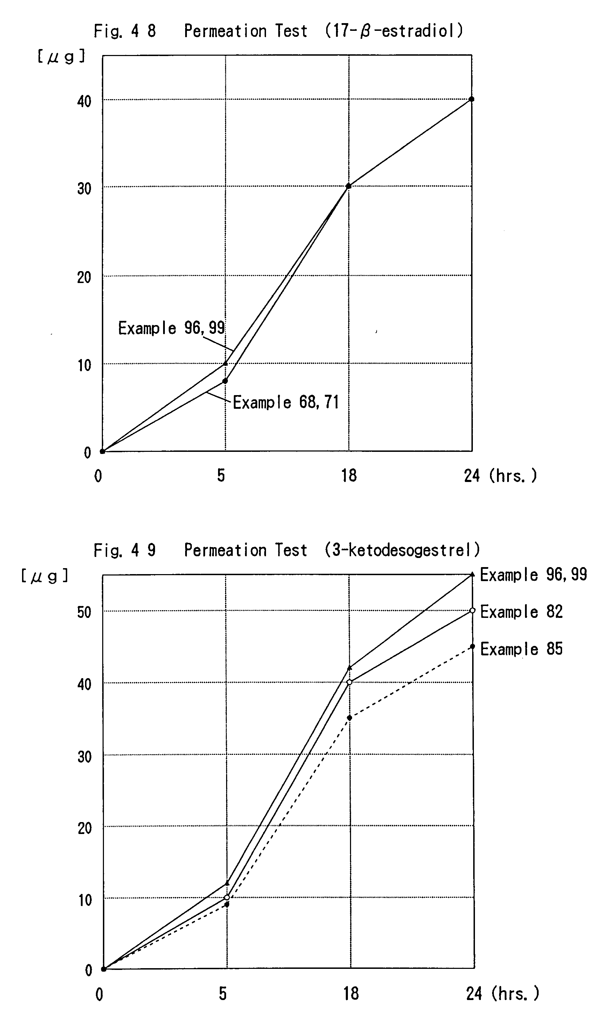

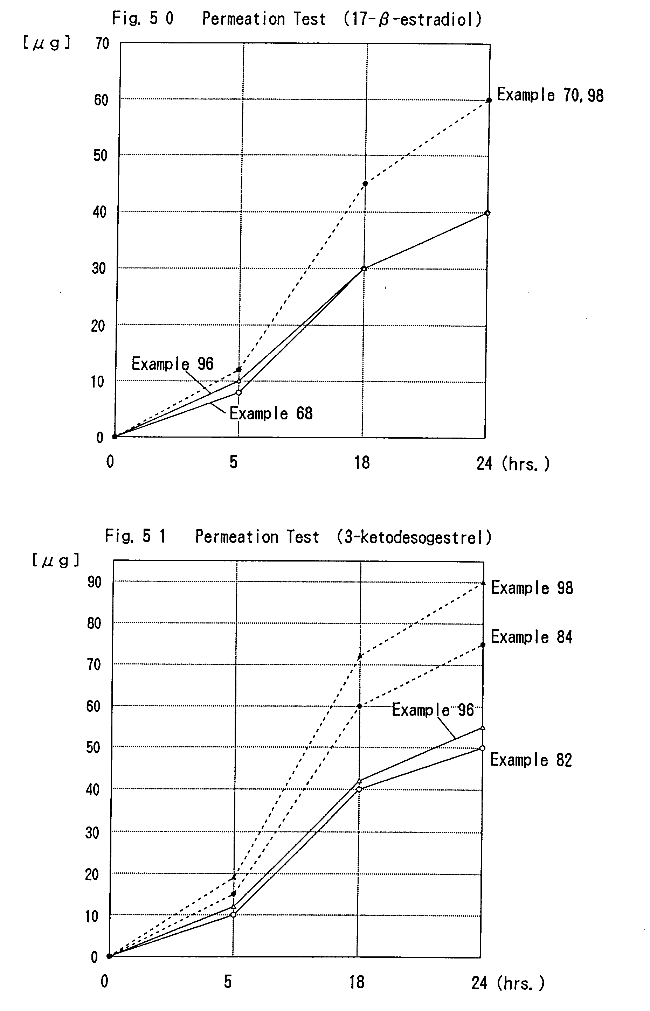

The influences of the constitutive components (polyacrylic acid, polyethylene glycol #400, propylene glycol, ethyl alcohol and isopropyl alcohol) or their concentrations in the base material on the percutaneous permeation are shown in Figs. 40 to 51.

### Examples 109 to 152:

### Production of Adhesives A to E:

Determined amounts (parts by weight) of the monomers as indicated in Table 12 and 400.0 parts by weight of ethyl acetate were fed into a separation flask equipped with a stirrer and a condenser and heated up to 60°C while stirring in nitrogen atmosphere. A solution prepared by dissolving 2.0 parts by weight of lauroyl peroxide in 100.0 parts by weight of cyclohexane was divided into 10 parts. One of these 10 parts was added to the separation flask and the polymerization was started. 5 hours after the initiation of the reaction, the remaining nine were added thereto one after another at intervals of one hour. After completion of the addition, the reaction was continued for 19 additional hours. To adjust the viscosity of the mixture, 50 parts by weight of ethyl acetate were added to the flask, five times in all, at intervals of 5 hours after the start of the reaction. After completion of the reaction, the flask was cooled, and ethyl acetate was added thereto in an amount that made the solid concentration to be 35 % by weight.

### Production of Adhesive F:

Pre-determined amounts (parts by weight) of the monomers as indicated in Table 12 and 256.0 parts by weight of ethyl acetate were fed into a separation flask equipped with a stirrer and a condenser and heated up to 70°C while stirring in nitrogen atmosphere. A solution prepared by dissolving 2.0 parts by weight of lauroyl peroxide in 100.0 parts by weight of cyclohexane was divided into 10 parts. One of these 10 parts was added to the separation flask and the polymerization was started. 5 hours after the initiation of the reaction, the remaining nine were added thereto one after another at intervals of one hour. After completion of the addition, the reaction was continued for 19 additional hours. To adjust the viscosity of the reaction system, 27 parts by weight of ethyl acetate were added to the flask, five times in all, at intervals of 5 hours after the start of the reaction. After completion of the reaction, the flask was cooled, and ethyl acetate was added thereto in an amount that made the solid concentration to be 50 % by weight.

### Production of Adhesive G:

100 parts by weight of natural rubber, 20 parts by weight of polybutene, 80 parts by weight of polyterpene resin (YS Resin PX1150, produced by Yasuhara Resin Co.) and 1000 parts by weight of cyclohexane were fed into a separation flask equipped with a stirrer and a condenser and dissolved by stirring for 96 hours at 25°C to obtain an adhesive having a solid content of 16.7 % by weight.

### Preparation of Adhesive Plasters:

Pre-determined amounts (parts by weight) of the adhesive, 3-ketodesogestrel, 17-β-estradiol, N-lauroylsarcosine, maleic acid, malic acid, capric acid, lauric acid diethanolamide and/or isopropyl myristate as indicated in Tables 13 to 16 below were fed into a dissolver and blended homogeneously. The resulting liquid mixture was coated on a silicone-treated polyethylene terephthalate film (thickness: 40 µm) and dried to form thereon an adhesive layer having a thickness of 60 µm. Next, this was transferred to a 50 µm-thick polyethylene terephthalate/ethylene-vinyl acetate copolymer laminate film on the vinyl acetate copolymer layer to obtain a percutaneous pharmaceutical preparation sample of the present invention.

**Table 12**

| Monomers | Adhesives | | | | | |
|---|---|---|---|---|---|---|
| | A | B | C | D | E | F |
| 2-Ethylhexyl Acrylate | 302.0 | 249.6 | 333.1 | 379.4 | 166.3 | 34.9 |
| Dodecyl Methacrylate | - | - | - | - | - | 48.3 |
| 2-Ethylhexyl Methacrylate | - | - | - | - | - | 301.0 |
| N-vinyl-2-pyrrolidone | 98.0 | 150.4 | 66.9 | 25.1 | 133.7 | - |
| 1,6-Hexane-glycol Di-methacrylate | 0.04 | 0.04 | 0.04 | 0.04 | 0.04 | 0.0384 |

**Table 13**

| | Example | | | | | | | |
|---|---|---|---|---|---|---|---|---|
| | 109 | 110 | 111 | 112 | 113 | 114 | 115 | 116 |
| Adhesive (solid content) | A | B | C | D | E | F | G | H |
| | 92 | 92 | 94 | 97 | 88 | 97 | 99 | 99 |
| Active ingredient 3-Ketodesogestrel | 8 | 8 | 6 | 3 | 12 | 3 | 1 | 1 |
| H: Silicone adhesive (Silascon 355, produced by Dow Corning Co.) | | | | | | | | |

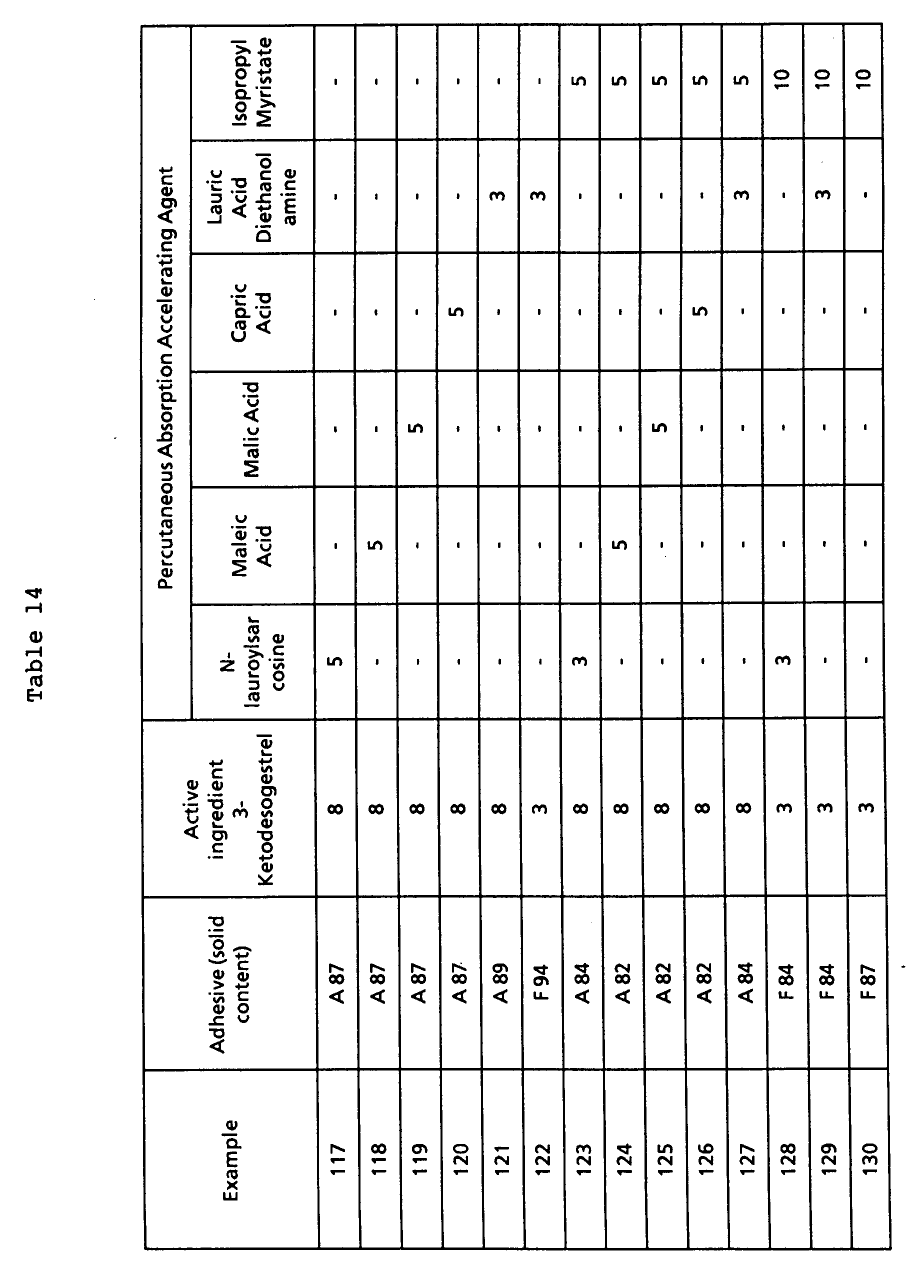

**Table 15**

| | Example | | | | | | | |
|---|---|---|---|---|---|---|---|---|
| | 131 | 132 | 133 | 134 | 135 | 136 | 137 | 138 |
| Adhesive (solid content) | A | B | C | D | E | F | G | H |
| | 88 | 88 | 92.8 | 96.4 | 85.6 | 96.4 | 98.8 | 98.8 |

| Active ingredient | | | | | | | | |
|---|---|---|---|---|---|---|---|---|
| 3-Ketodesogestrel | 6 | 6 | 3.6 | 1.8 | 7.2 | 1.8 | 0.6 | 0.6 |
| 17-β-estradiol | 6 | 6 | 3.6 | 1.8 | 7.2 | 1.8 | 0.6 | 0.6 |
| H: Silicone adhesive (Silascon 355, produced by Dow Corning Co. | | | | | | | | |

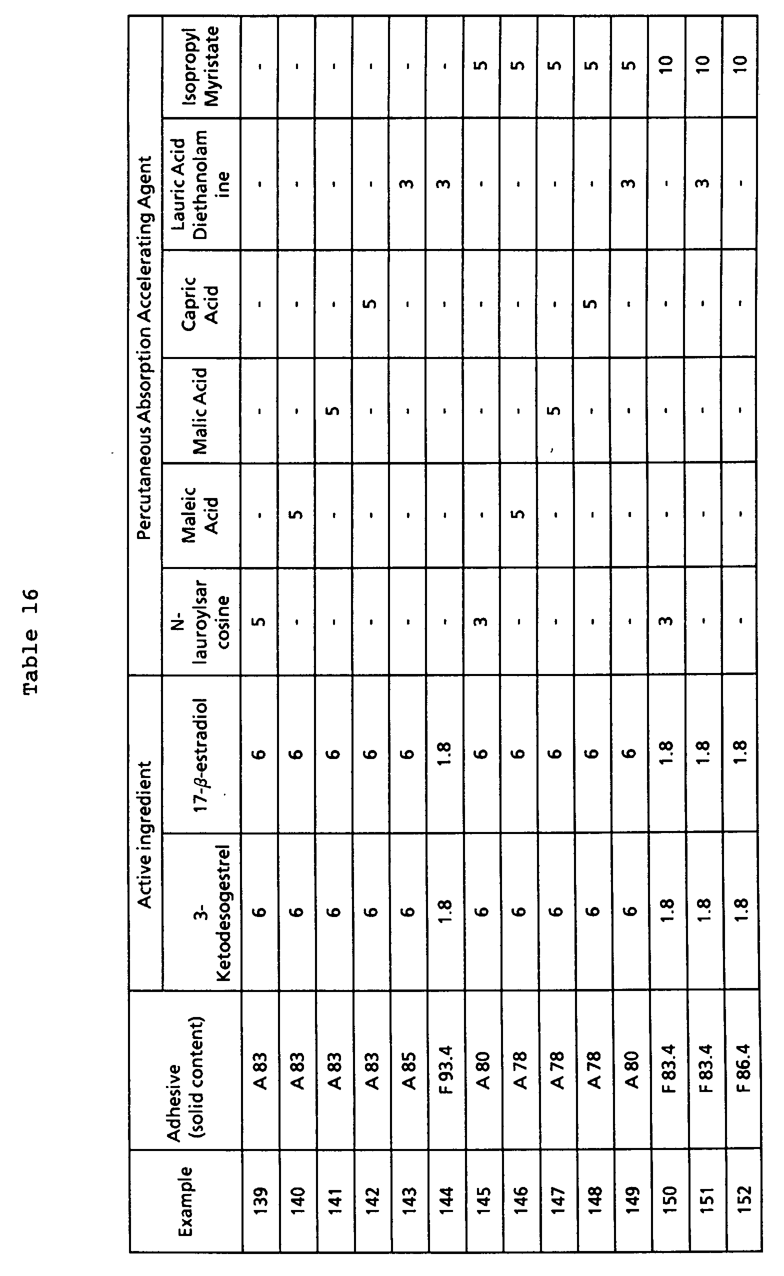

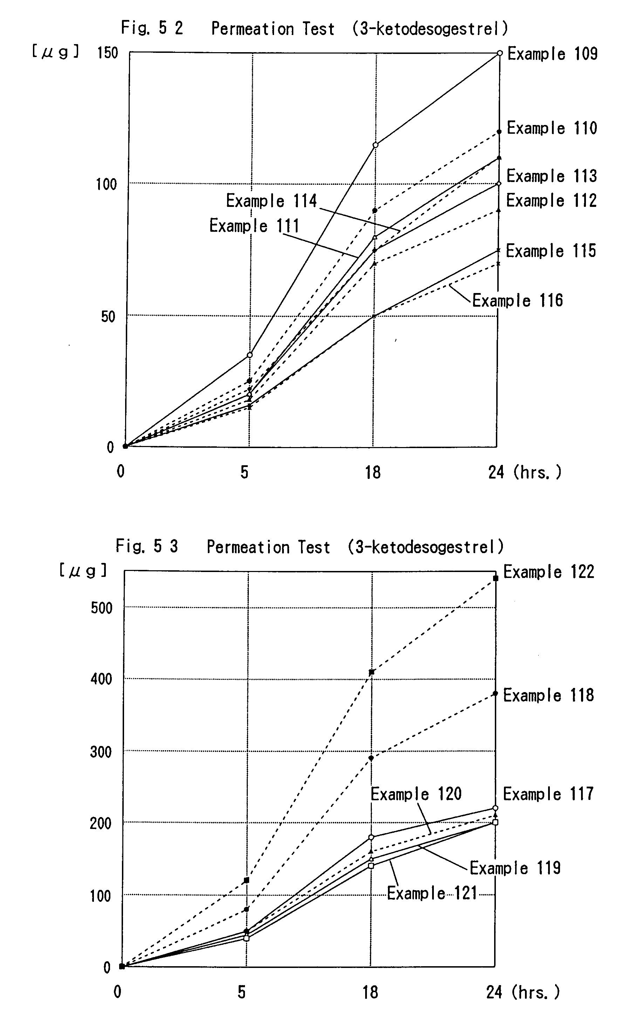

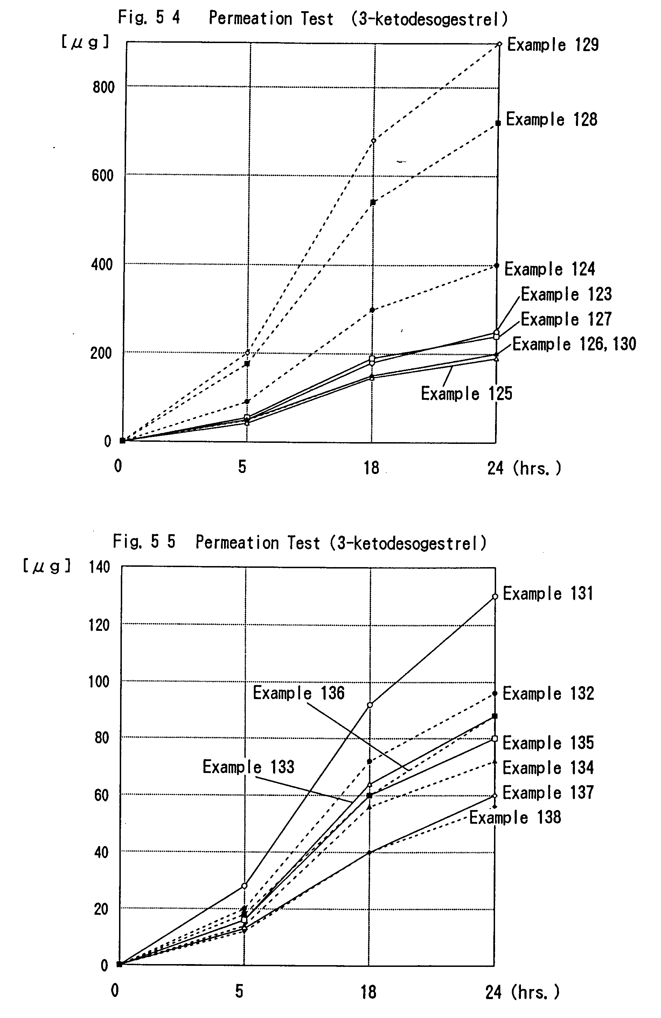

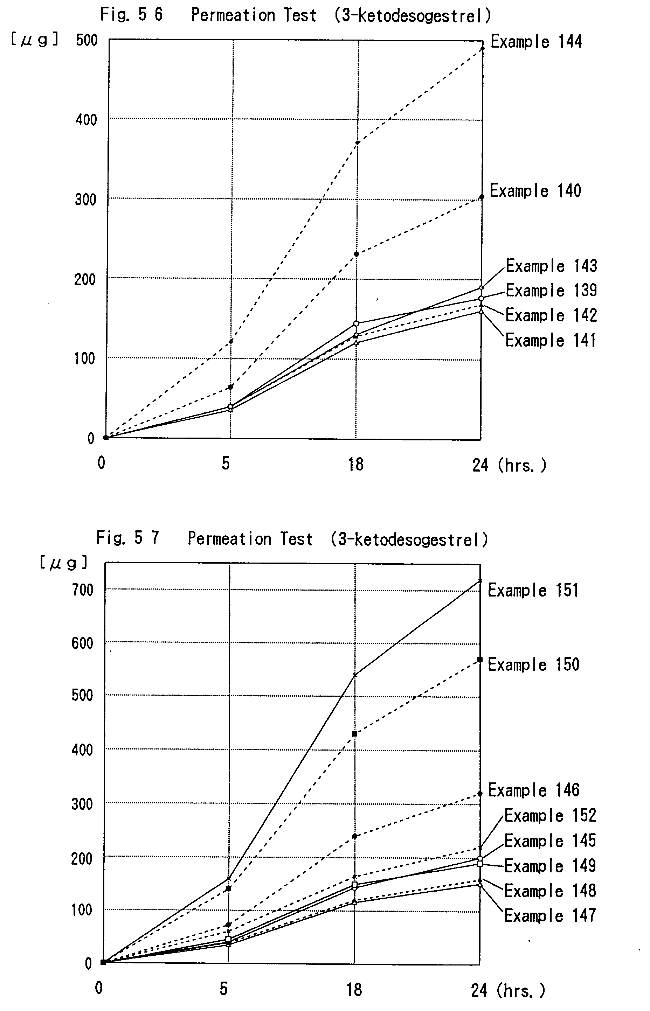

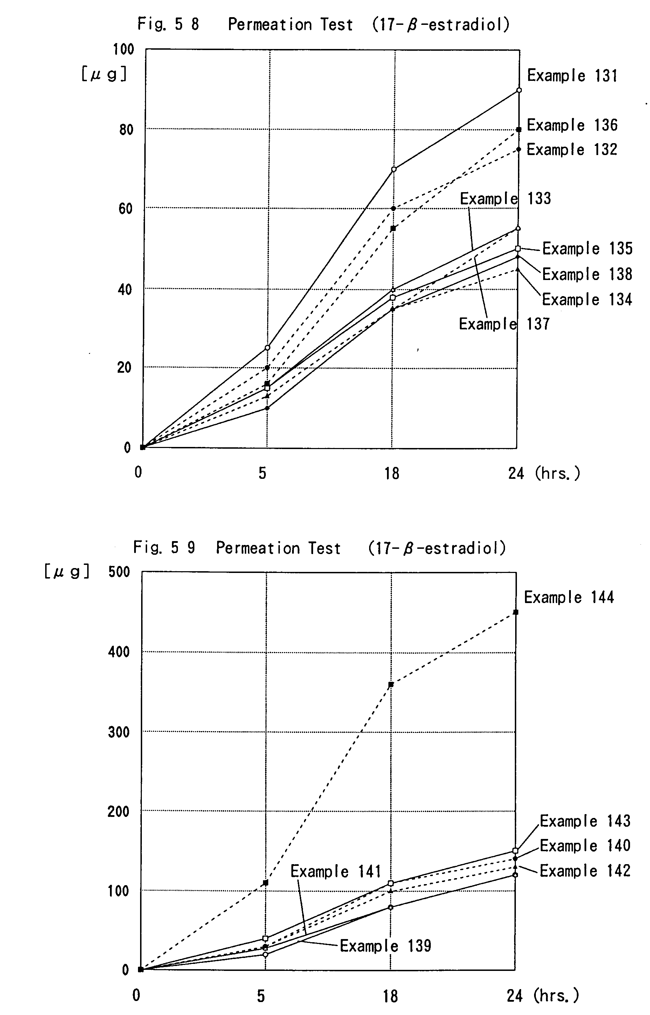

The pharmaceutical preparation samples thus obtained were subjected to the same permeation test as that in Example 1, using the diffusion cell 1 shown in Fig. 65. The structure of the diffusion cell used herein is the same as that in Example 1. In these examples, however, each percutaneous pharmaceutical preparation sample was cut into a circle (10 cm²) and attached to the center of the skin specimen 8 with the adhesive layer being kept in contact with the skin specimen 8. The test results are shown in Figs. 52 to 60.

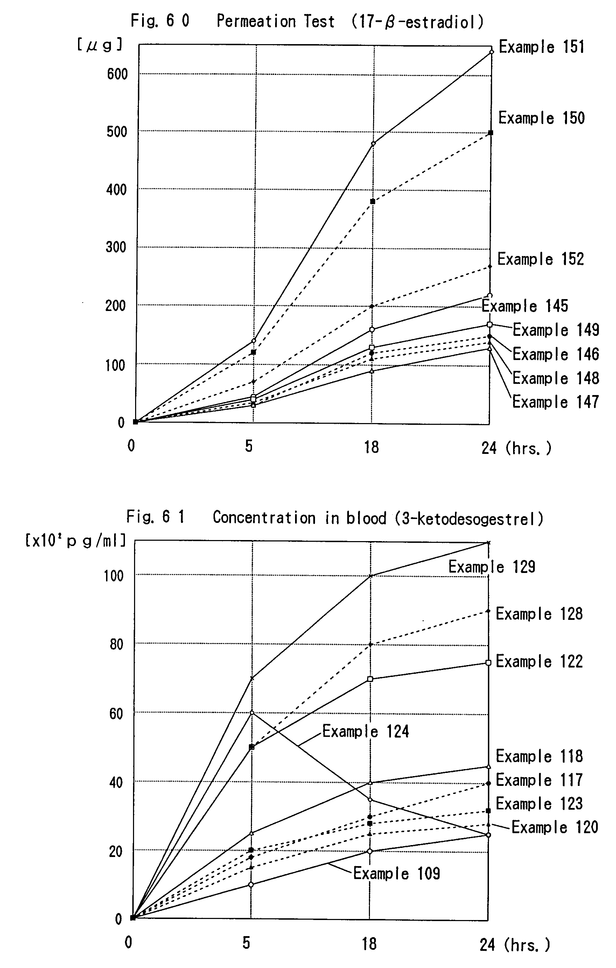

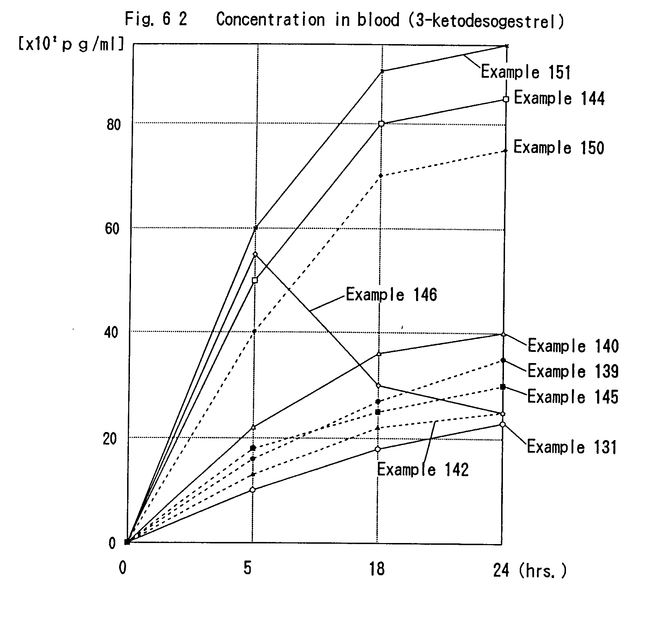

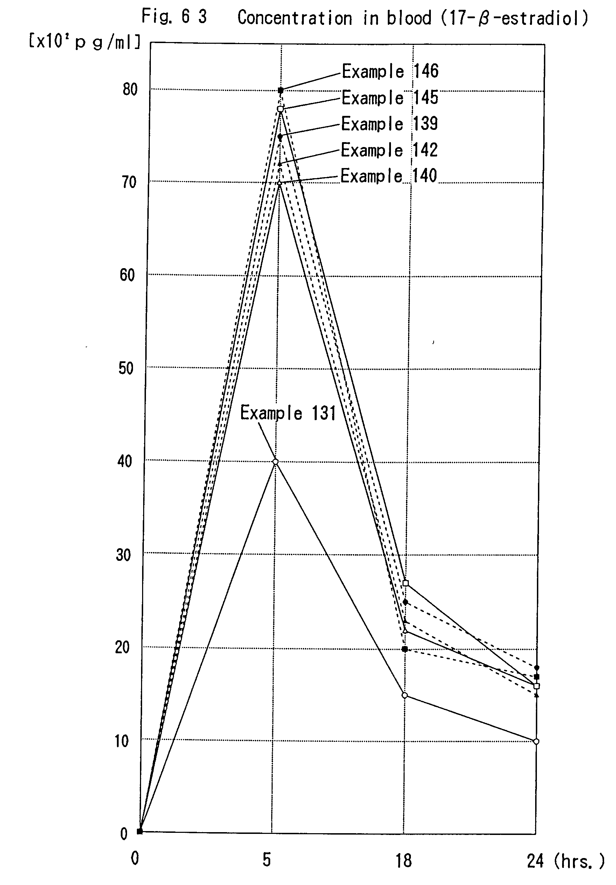

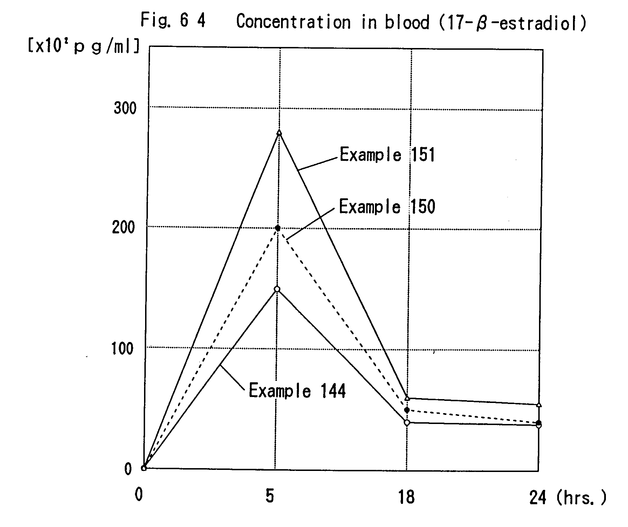

The pharmaceutical preparation sample obtained in each of Examples 109, 117, 118, 120, 122 to 124, 128, 129, 131, 139, 140, 142, 144 to 146, 150 and 151 was applied to rats in order to measure the concentration(s) of the active ingredient(s) in the blood. Precisely, the back of a Wister rat (7 weeks old, male) was completely shaven, and it was confirmed to be neither wounded nor inflamed. 24 hours after the shaving, the one inch-square pharmaceutical preparation sample was applied to the balded back. 5, 18 and 24 hours after the application, the blood was drawn. The concentration(s) of the active ingredient(s) in the blood was/were measured by radio-immunoassay. The results are shown in Figs. 61 to 64.

The pharmaceutical preparation samples (9 cm × 9 cm) obtained in Examples 109, 123, 124, 125, 126, 131, 145, 146, 147 and 148 were each wrapped under any one of the following wrapping conditions and stored at 25°C, 40°C and 60°C for 30 days. After this storage period, the ratio of the remaining amount of 3-ketodesogestrel (in percentage) to the initial content thereof was measured.
**Wrapping Condition 1:**
   A 12 µm-thick polyethylene terephthalate film and a 13 µm-thick polyethylene film were laminated on one surface of a 9 µm-thick aluminium film, while a 40 µm-thick polyethylene film was laminated on the other surface of the same, to form a laminate film. Two laminate films (10 cm × 10 cm) thus formed were heat-sealed, having the pharmaceutical preparation sample therebetween, in such a way that their polyethylene films faced each other.
**Wrapping Condition 2:**
   This is same as Condition 1 above, except that the sample was wrapped together with one disoxidant. (Ageless Z-20, product of Mitsubishi Gas Chemical Co.; having the ability of absorbing 20 cm³ of oxygen).
**Wrapping Condition 3:**
   This is same as Condition 1 above, except that the sample was wrapped together with 2.5 g of silica gel.

The amount of the remaining 3-ketodesogestrel (in percentage) was measured by extracting the stored sample with 50 ml of methanol for 24 hours at 25°C followed by subjecting the extract to high performance liquid chromatography. Each test was performed three samples (n=3) for each type of wrapping condition, and the averages were calculated.

The results are shown in Tables 17 and 18.

**Table 17**

| Example No. | Wrapping Condition | Fresh Sample | After storage at 25°C | After storage at 40°C | After storage at 60°C |
|---|---|---|---|---|---|
| 109 | 1 | 100 | 99 | 98 | 97.4 |
| | 2 | 100 | 100 | 100 | 100 |
| | 3 | 100 | 100 | 100 | 99 |
| 123 | 1 | 100 | 97 | 94.5 | 91 |
| | 2 | 100 | 100 | 100 | 100 |
| | 3 | 100 | 98 | 96 | 93 |
| 124 | 1 | 100 | 81 | 67.7 | 38.2 |
| | 2 | 100 | 98 | 94 | 91 |
| | 3 | 100 | 85 | 68 | 40 |
| 125 | 1 | 100 | 94 | 88.7 | 78 |
| | 2 | 100 | 99 | 98 | 96 |
| | 3 | 100 | 96 | 88.9 | 79 |
| 126 | 1 | 100 | 98 | 96.5 | 94 |
| | 2 | 100 | 100 | 100 | 100 |
| | 3 | 100 | 98 | 97 | 94 |

**Table 18**

| Example No. | Wrapping Condition | Fresh Sample | After storage at 25°C | After storage at 40°C | After storage at 60°C |
|---|---|---|---|---|---|
| 131 | 1 | 100 | 99 | 98 | 97.4 |
| | 2 | 100 | 100 | 100 | 100 |
| | 3 | 100 | 100 | 100 | 99 |
| 145 | 1 | 100 | 97 | 94.5 | 91 |
| | 2 | 100 | 100 | 100 | 100 |
| | 3 | 100 | 98 | 96 | 93 |
| 146 | 1 | 100 | 81 | 67.7 | 38.2 |
| | 2 | 100 | 98 | 94 | 91 |
| | 3 | 100 | 85 | 68 | 40 |
| 147 | 1 | 100 | 94 | 88.7 | 78 |
| | 2 | 100 | 99 | 98 | 96 |
| | 3 | 100 | 96 | 88.9 | 79 |
| 148 | 1 | 100 | 98 | 96.5 | 94 |
| | 2 | 100 | 100 | 100 | 100 |
| | 3 | 100 | 98 | 97 | 94 |

### INDUSTRIAL APPLICABILITY

The percutaneous pharmaceutical preparations of the present invention are easily produced and easily used, and they percutaneously release 3-ketodesogestrel or its 17-ester, and/or 17-β-estradiol or its ester uniformly over a prolonged period of time through the skin with stratum corneum.

Accordingly, the percutaneous pharmaceutical preparations of the present invention can be used efficaciously for contraception and also for relieving the symptoms of menopausal disorders, osteoporosis, emmeniopathy, etc.

## Claims

1. A percutaneous pharmaceutical preparation comprising a base material, one or more active ingredients selected from a group consisting of 3-ketodesogestrel and its 17-esters, and/or one or more active ingredients selected from a group consisting of 17-β-estradiol and its esters.

2. The percutaneous pharmaceutical preparation according to claim 1, in which the amount of one or more active ingredients selected from a group consisting of 3-ketodesogestrel and its 17-esters and/or one or more active ingredients selected from a group consisting of 17-β-estradiol and its esters is from 0.001 to 20 % by weight of the preparation.

3. The percutaneous pharmaceutical preparation according to claim 2, in which the amount of the active ingredient(s) is from 0.1 to 10 % by weight of the preparation.

4. The percutaneous pharmaceutical preparation according to any one of claims 1 to 3, in which the base material is composed of one or more selected from a group consisting of sodium alginate, gelatin, corn starch, tragacanth gum, methyl cellulose, hydroxyethyl cellulose, hydroxypropyl cellulose, carboxymethyl cellulose, sodium carboxymethyl cellulose, dextrin, carboxymethyl starch, polyvinyl alcohol, polyacrylic acid, polyacrylates, methoxyethylene-maleic anhydride copolymer, polyvinyl ether, polyvinyl pyrrolidone, colloidal aluminium silicate hydrate minerals, colloidal magnesium aluminium silicate hydrate minerals, bees wax, olive oil, cacao oil, sesame oil, soybean oil, camellia oil, peanut oil, beef tallow, lard, lanolin, vaseline, white vaseline, paraffins, liquid paraffin, plastibase, glycerin, ethylene glycol, polyethylene glycol, propylene glycol, polypropylene glycol, higher fatty acids, lower alcohols, higher alcohols and water, or a composition containing one or more of these.

5. The percutaneous pharmaceutical preparation according to any one of claims 1 to 4, which further contains one or more percutaneous absorption accelerating agents selected from a group consisting of N-acylsarcosines or their salts; monocarboxylic acids having from 8 to 14 carbon atoms or their salts; dicarboxylic acids having from 2 to 8 carbon atoms or their salts; hydroxydicarboxylic acids having from 3 to 8 carbon atoms; fatty acid amides, or reaction products of aliphatic monocarboxylic acid(s) having from 8 to 16 carbon atoms and mono- or di-ethanolamine(s); and esters of higher fatty acids, or reaction products of higher fatty acid(s) having from 10 to 18 carbon atoms and alcohol(s) having from 1 to 18 carbon atoms.

6. The percutaneous pharmaceutical preparation according to claim 5, in which the amount of the percutaneous absorption accelerating agent(s) is from 0.1 to 20 % by weight of the preparation.

7. The percutaneous pharmaceutical preparation according to claim 6, in which the amount of the percutaneous absorption accelerating agent(s) is from 3 to 10 % by weight of the preparation.

8. The percutaneous pharmaceutical preparation according to any one of claims 1 to 7, which is ointment.

9. The percutaneous pharmaceutical preparation according to claim 8, in which the base material comprises a polyacrylic acid or its salt, polyethylene glycol or propylene glycol, a lower alcohol and water.

10. The percutaneous pharmaceutical preparation according to claim 9, in which the amount of the polyacrylic acid or its salt is from 0.01 to 3 % by weight of the ointment, the amount of the polyethylene glycol or propylene glycol is from 5 to 45 % by weight of the same, the amount of the lower alcohol is from 20 to 65 % by weight of the same, and the amount of water is from 5 to 30 % by weight of the same.

11. The percutaneous pharmaceutical preparation according to claim 10, in which the amount of the polyacrylic acid or its salt is from 0.5 to 2.3 % by weight of the ointment, the amount of the polyethylene glycol or propylene glycol is from 10 to 44 % by weight of the same, the amount of the lower alcohol is from 25 to 64 % by weight of the same, and the amount of water is from 10 to 28 % by weight of the same.

12. The percutaneous pharmaceutical preparation according to claim 8, in which the percutaneous absorption accelerating agent is lauric acid diethanolamide.

13. The percutaneous pharmaceutical preparation according to claim 12, in which the amount of lauric acid diethanolamide is from 0.1 to 20 % by weight of the preparation.

14. The percutaneous pharmaceutical preparation according to claim 13, in which the amount of lauric acid diethanolamide is from 3 to 10 % by weight of the preparation.

15. A percutaneous pharmaceutical preparation comprising polyacrylic acid as base, lauric acid diethanolamide as percutaneous absorption accelerating agent, and an active ingredient.

16. The precutaneous pharmaceutical preparation of claim 15, wherein the active ingredient is a steroid.

17. The percutaneous pharmaceutical preparation of claim 15 or 16 wherein the active ingredient comprises an progestagenic compound.

18. The percutaneous pharmaceutical preparation of any one of claims 15-17, wherein the active ingredient comprises a 19-nor-testosterone derivative.

19. The percutaneous pharmaceutical preparation of claim 18, wherein the 19-nor-testosterone derivative is selected from desogestrel, 3-ketodesogestrel, levonorgestrel, gestodene, norgestrel, norgestrienone, noretistherone, norethindron, and norgestimate, or an ester thereof.

20. The percutaneous pharmaceutical preparation of claim 19, wherein the 19-nor-testosterone derivative is 3-ketodesogestrel or an ester thereof.

21. The percutaneous pharmaceutical preparation of any one of claims 17-20, whererin the active ingredient further comprises an estrogenic compound.

22. The percutaneous pharmaceutical preparation of claim 21, wherein the estrogenic compound is selected from ethynyl estradiol and β-estradiol, or an ester thereof.

23. The percutaneous pharmaceutical preparation of claim 20, wherein the preparation comprises 3-ketodesogestrel and β-estradiol, or an ester thereof.

24. The percutaneous pharmaceutical preparation of any one of claims 15-23, wherein the amount of lauric acid diethanolamide is from 0.1 to 20% by weight of the preparation.

25. The percutaneous pharmaceutical preparation of any one of claims 15-24, wherein the amount of lauric acid diethanolamide is from 3 to 10% by weight of the preparation.

26. The percutaneous pharmaceutical preparation of any one of claims 15-25, further comprising a backing layer and an adhesive base layer laminated on one surface of the backing layer, in which the adhesive base layer comprises an adhesive.

27. The percutaneous pharmaceutical preparation of claim 26, wherein the amount of the active ingredient is from 0.001 to 20% by weight of the adhesive base layer.

28. The percutaneous pharmaceutical preparation of claim 27, wherein the amount of the active ingredient is from 0.5 to 12% by weight of the adhesive base layer.

29. The percutaneous pharmaceutical preparation of any one of claims 26-28, wherein the adhesive is selected from acrylic adhesives, rubber adhesives, silicone adhesives and urethane adhesives.

30. The percutaneous pharmaceuical preparation of claim 29, wherein the adhesive is an acrylic adhesive, comprising a copolymer composed of monomers of alkyl (meth)acrylate(s) and copolymerizable vinyl compound(s).

31. The percutaneous pharmaceutical preparation of claim 30, wherein the acrylic adhesive comprises a copolymer composed of monomers of alkyl (meth)acrylate(s) and N-vinyl-2-pyrrolidone.

32. The percutaneous phamaceutical preparation of claim 31, wherein the acrylic adhesive comprises a copolymer composed of from 55 to 95% by weight of 2-ethylhexyl acrylate, from 5 to 45% by weight of N-vinyl-2-pyrrolidone, and from 0 to 0.5% by weight of polyfunctional monomer(s).

33. The percutaneous pharmaceutical preparation of claim 29, wherein the acrylic adhesive comprises a copolymer composed of plural alkyl (meth)acrylate monomers.

34. The percutaneous pharmaceutical preparation of claim 33, wherein the acrylic adhesive comprises a copolymer composed of from 65 to 90% by weight of 2-ethylhexyl methacrylate, from 5 to 30% by weight of 2-ethylhexyl acrylate, from 5 to 30% by weight of dodecyl methacrylate and from 0 to 0.5% by weight of polyfunctional monomer(s).

35. The percutaneous pharmaceutical preparation of any one of claims 15-34 which is wrapped with a wrapping container having an oxygen permeability of 20 cm³/m².atm.24h (25°C) or less and selected from aluminium sheeting, polyvinylidene chloride film, polyvinylidene chloride-polyethylane laminate film, polyvinylidene chloride-polypropylene laminate film, polyvinylidene chloride-polyvinyl chlorode laminate film, polyvinyl alcohol film and nylon film.
